# EUROPEAN PATENT APPLICATION

(11) **EP 4 151 721 A1**
(43) Date of publication of application: **22.03.2023**
(21) Application number: 20935084.2
(22) Date of filing: 15.05.2020
(51) Int. Cl.: C12N 5/0783, C12N 5/10, C12N 15/113, C12N 15/09, C07K 14/705, A61K 35/17, A61P 35/00, A61P 31/00

(54) **T CELL, PREPARATION METHOD FOR SAME, AND APPLICATIONS THEREOF**

(71) Applicant: Benethera (Beijing) Biotechnology Co., Ltd., Beijing 100085 (CN)
(72) Inventor: SHI, Yan, Beijing 100084 (CN); MENG, Junchen, Beijing 100084 (CN); WANG, Xiaobo, Beijing 100084 (CN); GENG, Shuang, Beijing 100084 (CN)
(74) Representative: Murgitroyd & Company
(86) International application number: PCT/CN2020/090639
(87) International publication number: WO 2021/227056

(57) **Abstract**

Provided are a T cell deleting the Ryr2 gene and a preparation method. Also provided are an Ryr2 antagonist, an Ryr2 overexpressing T cell, a method and applications for regulating Ryr2 expression, regulating basal Ca²⁺ oscillation, regulating m-Calpain activity, and increasing bonding strength between T cells and DC cells, applications of a reagent implementing the functions in preparing a medicament for treating an infectious disease, inflammation, or tumor, and a method for transforming Tconv cells into being functionally similar to Treg cells.

## Description

### TECHNICAL FIELD

The present invention relates to the technical field of biotechnology, and particularly to a T cell, a method for preparing the same and use thereof, and use of a reagent for regulating Ryr2 expression, a reagent for regulating basal Ca²⁺ oscillation, a reagent for regulating m-calpain activity or a reagent for regulating binding strength of a T cell to a DC cell in preparing a medicament for treating an infectious disease, an inflammation or a tumor.

### BACKGROUND

Regulatory T cells (Tregs) are a functional subpopulation of suppressor T cells. Natural Treg cells were first reported in 1995 by Sakaguchi et al., which account for about 5%-10% of peripheral blood CD4+ T cells. FoxP3 is a marker of the natural Tregs. Regulatory T cells can be divided into two categories: natural Tregs (n Tregs) and acquired Tregs (a Tregs).

The suppression mechanism of Treg cells is a research hotspot. The mechanisms disclosed in the reference "Past, Present, and Future of Regulatory T Cell Therapy in Transplantation and Autoimmunity" (Romano et al., Frontiers in Immunology, 2019), including T-cell lysis, surface protein extraction, local generation of adenosine and the like, all require Treg binding. In recent years, a series of research reports have shifted the focus to direct inhibition of dendritic cells. The main reasons are that: in one aspect, Treg cells far exceed dendritic cells in the number of CD4+ T cells, but Treg cells still predominate in number as compared with dendritic cells, with a ratio of about 2:1 *in vivo,* which infers a more rational and fair use of inhibitory capacity. Secondly, direct binding of DCs/Tregs is common both *in vivo* and *in vitro.* Although this constraint is considered as a suppression of the DCs, there is no consensus on the exact way of operation. The Sakaguchi's team proposed in reference "Trans-endocytosis of CD80 and CD86: a molecular basis for the cell-extrinsic function of CTLA-4" (Qureshi et al., Science 332, 2011) that this is mainly realized by endocytosis of CTLA-4 expressed on Tregs. In a recent paper, Shevach proposed that MHC class II extraction plays an important role in inhibiting DCs in antigen presenting.

However, the inventors believe that the binding between Tregs and DCs is mediated by LFA-1 and ICAM-1, and the binding itself is so exuberantly strong that the DC cytoskeleton is seriously distorted and cannot support antigen presentation to other T cells (see "Strong adhesion by regulatory T cells induces dendritic cell cytoskeletal polarization and contact-dependent lethargy" (Chen et al., The Journal of experimental medicine, 2017)). These differences may be resolved over time, resulting in a more accurate and comprehensive image of how Tregs inhibits DCs through contact. However, in order to continue the discussion, there is a basic question that needs to be answered: why Tregs bind to DCs together with such a strong force, and how this excessive force affects DCs.

Ryr2 is a calcium release channel on endoplasmic reticulum/sarcoplasmic reticulum (ER/SR), and the prior art discloses Ryr2 gene is related to sudden cardiac death, arrhythmia, coronary heart disease, for example,
non-patent literature "Sudden Cardiac Death Caused by Ryr2 Gene Mutations: a Report of Pedigree Analysis" (Tong Shen et al., Cardiovascular Disease Journal of Integrated Traditional Chinese and Western Medicine, February, 2019) discloses that carrying the missense mutation c.G4107C on the Ryr2 gene causes symptoms such as exertional dyspnea and chest pain, i.e., the missense mutation on the Ryr2 gene may be related to sudden cardiac death;
non-patent literature "Effects of CASQ2 and Ryr2 on Diacetylmorphine-Induced Arrhythmia in Cardiac Myocytes" (Xiayun Hu et al., Journal of Xinjiang Medical University, March, 2019) discloses the relationship between Ryr2 and calcium channels and confirms that the Ryr2 gene is involved in the process of abnormal calcium channels in cardiac myocytes and arrhythmia;
non-patent literature "Theoretical Discussion on the Correlation Between the Ryr2 Gene Expression and the Incidence of Coronary Heart Disease with Qi Deficiency Disorder" (Yuexiang Ma et al., Information on Traditional Chinese Medicine, 2012) discloses that Ryr2 dysfunction can cause the decline of heart function, which is consistent with heart governing blood and vessels in traditional Chinese medicine, wherein heart *qi* is the basic power for promoting blood circulation, and the heart-*qi* deficiency causes the decline of heart function; the Ryr2 gene expression can further reveal the essence of the heart-*qi* deficiency disorder; and
non-patent literature "Inhibitory Effect of Carvedilol on Ryr2-Mediated Spontaneous Calcium Oscillation" (Jianmin Xiao et al., Chinese Journal of Pathophysiology, 2013) discloses that carvedilol can inhibit Ryr2-mediated spontaneous calcium oscillation and indicates that carvedilol is superior to other β-blockers in reducing the mortality of heart failure.

However, the prior art does not disclose the correlation of Ryr2 with T cells and infectious diseases, inflammation or tumors.

### SUMMARY

In order to make up for the gap in the prior art, the present invention found a regulation switch for the Treg cell function-Ryr2, acquired a technique that can reduce Ryr2 expression through FoxP3 overexpression, and specifically determined the target position. Meanwhile, a large number of experiments demonstrated that the knock down or knock out of the Ryr2 in the T cells enables the CD4+ T cell pool to become immunosuppressive cells, the obtained T cells have functions similar to those of Treg cells. Furthermore, the treatment of viral infections, asthma, allergies, colitis and tumors demonstrated good effects and the autoimmunity of the psoriasis mouse-related system were recovered through the regulation of Ryr2.

Specifically, in a first aspect of the present invention, provided is a T cell, wherein Ryr2 gene is deleted or FoxP3 is overexpressed in the T cell.

Preferably, at least exon 7 of the Ryr2 gene is deleted in the T cell.

Preferably, at least a guanine-rich sequence in the Ryr2 gene is deleted in the T cell. Preferably, the guanine-rich sequence is located within first 200 bp from a start codon of the Ryr2 gene.

Preferably, the guanine-rich sequence is a nucleotide sequence comprising at least 4 guanines (G), more preferably, a nucleotide comprising at least 4-20 guanines (G), and in one specific embodiment of the present invention, comprising at least 4, 5, 6, 7, 8, 9, 10, 11, 12, 13, 14 or 15 guanines or the like.

Preferably, the guanine-rich sequence comprises GCAGGGG.

Preferably, the T cell is selected from a cytotoxic T cell, a helper T cell, a regulatory/suppressor T cell (Treg) or a memory T cell.

In one specific embodiment of the present invention, the T cell is a Tconv cell.

In a second aspect of the present invention, provided is a method for preparing the T cell described herein, wherein the method is selected from shRNA, siRNA, CRISPR/Cas9, zinc finger nuclease technology, transcription activator-like effector nuclease technology or meganuclease. Preferably, the shRNA is set forth in SEQ ID NO: 5 or 6.

In a third aspect of the present invention, provided is an shRNA, wherein the shRNA knocks down Ryr2 gene of a T cell, thus reducing basal Ca²⁺ oscillation in the T cell, reducing m-Calpain activity in the T cell, improving binding strength T cell to DC cells, allowing the T cell to have functions of immunosuppressive cells or functions of treating an infectious disease, an inflammation or a tumor.

Preferably, the shRNA is set forth in SEQ ID NO: 5 or 6.

In a fourth aspect of the present invention, provided is a T cell, wherein Ryr2 is overexpressed in the T cell.

Preferably, the T cell is selected from a cytotoxic T cell, a helper T cell, a regulatory/suppressor T cell (Treg) or a memory T cell.

In one specific embodiment of the present invention, the T cell is a Treg cell.

In a fifth aspect of the present invention, provided is use of a reagent for regulating FoxP3 expression, a reagent for regulating Ryr2 expression, a reagent for regulating basal Ca²⁺ oscillation, a reagent for regulating m-calpain activity or a reagent for regulating binding strength of a T cell to a DC cell in preparing a medicament for treating an infectious disease, an inflammation or a tumor.

Preferably, the infectious disease is selected from a bacterial infection, a viral infection or a fungal infection; more preferably selected from a viral infection, pneumonia with septic shock, peritonitis, bacteremia, sepsis or septicemia; the viral infection is selected from an acute viral infection or a chronic viral infection, and the virus is preferably selected from influenza virus, parainfluenza virus, herpes virus (e.g., HSV-1, EBV), measles virus, vesicular stomatitis virus, hepatitis B virus, hepatitis C virus, human immunodeficiency virus, lymphocytic choriomeningitis virus or human papilloma virus.

Preferably, the inflammation may be an inflammation occurring in any tissue, wherein the tissue includes, but is not limited to, adrenal gland, adrenal medulla, anus, appendix, bladder, blood, bone, bone marrow, brain, mammary gland, cecum, central nervous system (including or excluding brain), cerebellum, cervix, colon, duodenum, endometrium, epithelial cells (e.g., renal epithelial cells), gallbladder, esophagus, glial cells, heart, ileum, jejunum, kidney, lacrimal gland, larynx, liver, lung, lymph, lymph node, lymphoblasts, maxilla, mediastinum, mesenterium, myometrium, nasopharynx, omentum, oral cavity, ovary, pancreas, parotid gland, peripheral nervous system, peritoneum, pleura, prostate, salivary gland, sigmoid colon, skin, small intestine, soft tissue, spleen, stomach, testis, thymus, thyroid, tongue, tonsil, trachea, uterus, vulva, white blood cells. More preferably, the inflammation is selected from systemic lupus erythematosus, rheumatoid arthritis, psoriatic arthritis, scleroderma, asthma, atopic dermatitis, organ-specific inflammatory diseases, allergies (e.g., allergic rhinitis), folliculitis, tonsillitis, pneumonia, hepatitis, nephritis, acne, autoimmune diseases, chronic prostatitis, glomerulonephritis, hypersensitivity reactions, colitis, inflammatory bowel diseases, pelvic inflammatory disease, reperfusion injury, transplantation rejection, vasculitis or interstitial cystitis.

Preferably, the tumor may be any adverse cell proliferation (or any disease that manifests itself as adverse cell proliferation) or neoplasm, or increased predisposition or risk for adverse cell proliferation, neoplasm or a tumor. The tumor may be benign or malignant, and may also be primary or secondary (metastatic). The neoplasm may be any abnormal growth or proliferation of cells and may be located in any tissue. Examples of the tissue include adrenal gland, adrenal medulla, anus, appendix, bladder, blood, bone, bone marrow, brain, mammary gland, cecum, central nervous system (including or excluding brain), cerebellum, cervix, colon, duodenum, endometrium, epithelial cells (e.g., renal epithelial cells), gallbladder, esophagus, glial cells, heart, ileum, jejunum, kidney, lacrimal gland, larynx, liver, lung, lymph, lymph node, lymphoblasts, maxilla, mediastinum, mesenterium, myometrium, nasopharynx, omentum, oral cavity, ovary, pancreas, parotid gland, peripheral nervous system, peritoneum, pleura, prostate, salivary gland, sigmoid colon, skin, small intestine, soft tissue, spleen, stomach, testis, thymus, thyroid, tongue, tonsil, trachea, uterus, vulva or white blood cells. More preferably, the tumor is selected from prostate cancer, breast cancer, liver cancer, glioma (e.g., neuroglioma), intestinal cancer, cervical cancer, non-small cell lung cancer, lung cancer, pancreatic cancer, gastric cancer, bladder cancer, skin cancer, striated muscle cancer, tongue squamous carcinoma, nasopharyngeal cancer, ovarian cancer, placental choriocarcinoma, lymphoma (e.g., non-Hodgkin's lymphoma, Hodgkin's lymphoma or cutaneous T-cell lymphoma), leukemia, rectal adenocarcinoma, medulloblastoma, meningioma, neurofibroma (e.g., neurofibrosarcoma), ependymoma, schwannoma, astrocytoma, melanoma, mesothelioma, myeloma, chronic granulocytic leukaemia, acute myelogenous leukemia, myelodysplastic syndrome, chronic lymphocytic leukemia, epidermoid cancer, colon cancer, thymus cancer, blood cancer, head or neck cancer or oropharyngeal cancer.

In a sixth aspect of the present invention, provided is use of a reagent for regulating FoxP3 expression, a reagent for regulating Ryr2 expression, a reagent for regulating basal Ca²⁺ oscillation, a reagent for regulating m-calpain activity, a reagent for regulating binding strength of a T cell to a DC cell, or the T cell described herein in treating an infectious disease, an inflammation or a tumor.

In a seventh aspect of the present invention, provided is use of a reagent for regulating FoxP3 expression, a reagent for regulating Ryr2 expression, a reagent for regulating basal Ca²⁺ oscillation, a reagent for regulating m-calpain activity, a reagent for regulating binding strength of a T cell to a DC cell, or the T cell described herein in preparing a medicament for treating an infectious disease, an inflammation or a tumor.

In an eighth aspect of the present invention, provided is use of regulation of FoxP3 expression, regulation of Ryr2 expression, regulation of basal Ca²⁺ oscillation, regulation of m-calpain activity, regulation of binding strength of a T cell to a DC cell, or the T cell described herein in treating an infectious disease, an inflammation or tumor, or in preparing a medicament for treating an infectious disease, an inflammation or a tumor.

Preferably, the infectious disease is selected from a bacterial infection, a viral infection or a fungal infection, more preferably selected from a viral infection, pneumonia with septic shock, peritonitis, bacteremia, sepsis or septicemia; the viral infection is selected from an acute viral infection or a chronic viral infection, and the virus is preferably selected from influenza virus, parainfluenza virus, herpes virus (e.g., HSV-1, EBV), measles virus, vesicular stomatitis virus, hepatitis B virus, hepatitis C virus, human immunodeficiency virus, lymphocytic choriomeningitis virus or human papilloma virus.

Preferably, the inflammation may be an inflammation occurring in any tissue, wherein the tissue includes, but is not limited to, adrenal gland, adrenal medulla, anus, appendix, bladder, blood, bone, bone marrow, brain, mammary gland, cecum, central nervous system (including or excluding brain), cerebellum, cervix, colon, duodenum, endometrium, epithelial cells (e.g., renal epithelial cells), gallbladder, esophagus, glial cells, heart, ileum, jejunum, kidney, lacrimal gland, larynx, liver, lung, lymph, lymph node, lymphoblasts, maxilla, mediastinum, mesenterium, myometrium, nasopharynx, omentum, oral cavity, ovary, pancreas, parotid gland, peripheral nervous system, peritoneum, pleura, prostate, salivary gland, sigmoid colon, skin, small intestine, soft tissue, spleen, stomach, testis, thymus, thyroid, tongue, tonsil, trachea, uterus, vulva or white blood cells. More preferably, the inflammation is selected from systemic lupus erythematosus, rheumatoid arthritis, psoriatic arthritis, scleroderma, asthma, atopic dermatitis, organ-specific inflammatory diseases, allergies (e.g., allergic rhinitis), folliculitis, tonsillitis, pneumonia, hepatitis, nephritis, acne, autoimmune diseases, chronic prostatitis, glomerulonephritis, hypersensitivity reactions, colitis, inflammatory bowel diseases, pelvic inflammatory disease, reperfusion injury, transplantation rejection, vasculitis or interstitial cystitis.

Preferably, the tumor may be any adverse cell proliferation (or any disease that manifests itself as adverse cell proliferation) or neoplasm, or increased predisposition or risk for adverse cell proliferation, neoplasm or a tumor. The tumor may be benign or malignant, and may also be primary or secondary (metastatic). The neoplasm may be any abnormal growth or proliferation of cells and may be located in any tissue. Examples of the tissue include adrenal gland, adrenal medulla, anus, appendix, bladder, blood, bone, bone marrow, brain, mammary gland, cecum, central nervous system (including or excluding brain), cerebellum, cervix, colon, duodenum, endometrium, epithelial cells (e.g., renal epithelial cells), gallbladder, esophagus, glial cells, heart, ileum, jejunum, kidney, lacrimal gland, larynx, liver, lung, lymph, lymph node, lymphoblasts, maxilla, mediastinum, mesenterium, myometrium, nasopharynx, omentum, oral cavity, ovary, pancreas, parotid gland, peripheral nervous system, peritoneum, pleura, prostate, salivary gland, sigmoid colon, skin, small intestine, soft tissue, spleen, stomach, testis, thymus, thyroid, tongue, tonsil, trachea, uterus, vulva or white blood cells. More preferably, the tumor is selected from prostate cancer, breast cancer, liver cancer, glioma (e.g., neuroglioma), intestinal cancer, cervical cancer, non-small cell lung cancer, lung cancer, pancreatic cancer, gastric cancer, bladder cancer, skin cancer, striated muscle cancer, tongue squamous carcinoma, nasopharyngeal cancer, ovarian cancer, placental choriocarcinoma, lymphoma (e.g., non-Hodgkin's lymphoma, Hodgkin's lymphoma or cutaneous T-cell lymphoma), leukemia, rectal adenocarcinoma, medulloblastoma, meningioma, neurofibroma (e.g., neurofibrosarcoma), ependymoma, schwannoma, astrocytoma, melanoma, mesothelioma, myeloma, chronic granulocytic leukaemia, acute myelogenous leukemia, myelodysplastic syndrome, chronic lymphocytic leukemia, epidermoid cancer, colon cancer, thymus cancer, blood cancer, head or neck cancer or oropharyngeal cancer.

In a ninth aspect of the present invention, provided is use of a reagent for improving FoxP3 expression, a reagent for reducing Ryr2 expression, a reagent for reducing basal Ca²⁺ oscillation, a reagent for reducing m-calpain activity, a reagent for improving binding strength of a T cell to a DC cell, or the T cell with Ryr2 gene deletion described above in preparing a medicament for treating an infectious disease or an inflammation.

In a tenth aspect of the present invention, provided is use of a reagent for improving FoxP3 expression, a reagent for reducing Ryr2 expression, a reagent for reducing basal Ca²⁺ oscillation, a reagent for reducing m-calpain activity, a reagent for improving binding strength of a T cell to a DC cell, or the T cell with Ryr2 gene deletion described above in treating an infectious disease or an inflammation.

In an eleventh aspect of the present invention, provided is use of improvement of FoxP3 expression, reduction of Ryr2 expression, reduction of basal Ca²⁺ oscillation, reduction of m-calpain activity, improvement of binding strength of a T cell to a DC cell in treating an infectious disease or an inflammation, or preparing a medicament for treating an infectious disease or an inflammation.

Preferably, the infectious disease is selected from a bacterial infection, a viral infection or a fungal infection, more preferably selected from a viral infection, pneumonia with septic shock, peritonitis, bacteremia, sepsis or septicemia; the viral infection is selected from an acute viral infection or a chronic viral infection, and the virus is preferably selected from influenza virus, parainfluenza virus, herpes virus (e.g., HSV-1, EBV), measles virus, vesicular stomatitis virus, hepatitis B virus, hepatitis C virus, human immunodeficiency virus, lymphocytic choriomeningitis virus or human papilloma virus.

Preferably, the inflammation may be an inflammation occurring in any tissue, wherein the tissue includes, but is not limited to, adrenal gland, adrenal medulla, anus, appendix, bladder, blood, bone, bone marrow, brain, mammary gland, cecum, central nervous system (including or excluding brain), cerebellum, cervix, colon, duodenum, endometrium, epithelial cells (e.g., renal epithelial cells), gallbladder, esophagus, glial cells, heart, ileum, jejunum, kidney, lacrimal gland, larynx, liver, lung, lymph, lymph node, lymphoblasts, maxilla, mediastinum, mesenterium, myometrium, nasopharynx, omentum, oral cavity, ovary, pancreas, parotid gland, peripheral nervous system, peritoneum, pleura, prostate, salivary gland, sigmoid colon, skin, small intestine, soft tissue, spleen, stomach, testis, thymus, thyroid, tongue, tonsil, trachea, uterus, vulva or white blood cells. More preferably, the inflammation is selected from systemic lupus erythematosus, rheumatoid arthritis, psoriatic arthritis, scleroderma, asthma, atopic dermatitis, organ-specific inflammatory diseases, allergies (e.g., allergic rhinitis), folliculitis, tonsillitis, pneumonia, hepatitis, nephritis, acne, autoimmune diseases, chronic prostatitis, glomerulonephritis, hypersensitivity reactions, colitis, inflammatory bowel diseases, pelvic inflammatory disease, reperfusion injury, transplantation rejection, vasculitis or interstitial cystitis.

In a twelfth aspect of the present invention, provided is use of a reagent for reducing FoxP3 expression, a reagent for improving Ryr2 expression, a reagent for improving basal Ca²⁺ oscillation, a reagent for improving m-calpain activity, a reagent for reducing binding strength of a T cell to a DC cell, or the T cell overexpressing Ryr2 described above in preparing a medicament for treating a tumor.

In a thirteenth aspect of the present invention, provided is use of a reagent for reducing FoxP3 expression, a reagent for improving Ryr2 expression, a reagent for improving basal Ca²⁺ oscillation, a reagent for improving m-calpain activity, a reagent for reducing binding strength of a T cell to a DC cell, or the T cell overexpressing Ryr2 described above in treating a tumor.

In a fourteenth aspect of the present invention, provided is use of reduction of FoxP3 expression, improvement of Ryr2 expression, improvement of basal Ca²⁺ oscillation, improvement of m-calpain activity, reduction of binding strength of a T cell to a DC cell in treating a tumor, or preparing a medicament for treating a tumor.

Preferably, the tumor may be any adverse cell proliferation (or any disease that manifests itself as adverse cell proliferation) or neoplasm, or increased predisposition or risk for adverse cell proliferation, neoplasm or a tumor. The tumor may be benign or malignant, and may also be primary or secondary (metastatic). The neoplasm may be any abnormal growth or proliferation of cells and may be located in any tissue. Examples of the tissue include adrenal gland, adrenal medulla, anus, appendix, bladder, blood, bone, bone marrow, brain, mammary gland, cecum, central nervous system (including or excluding brain), cerebellum, cervix, colon, duodenum, endometrium, epithelial cells (e.g., renal epithelial cells), gallbladder, esophagus, glial cells, heart, ileum, jejunum, kidney, lacrimal gland, larynx, liver, lung, lymph, lymph node, lymphoblasts, maxilla, mediastinum, mesenterium, myometrium, nasopharynx, omentum, oral cavity, ovary, pancreas, parotid gland, peripheral nervous system, peritoneum, pleura, prostate, salivary gland, sigmoid colon, skin, small intestine, soft tissue, spleen, stomach, testis, thymus, thyroid, tongue, tonsil, trachea, uterus, vulva or white blood cells. More preferably, the tumor is selected from prostate cancer, breast cancer, liver cancer, glioma (e.g., neuroglioma), intestinal cancer, cervical cancer, non-small cell lung cancer, lung cancer, pancreatic cancer, gastric cancer, bladder cancer, skin cancer, striated muscle cancer, tongue squamous carcinoma, nasopharyngeal cancer, ovarian cancer, placental choriocarcinoma, lymphoma (e.g., non-Hodgkin's lymphoma, Hodgkin's lymphoma or cutaneous T-cell lymphoma), leukemia, rectal adenocarcinoma, medulloblastoma, meningioma, neurofibroma (e.g., neurofibrosarcoma), ependymoma, schwannoma, astrocytoma, melanoma, mesothelioma, myeloma, chronic granulocytic leukaemia, acute myelogenous leukemia, myelodysplastic syndrome, chronic lymphocytic leukemia, epidermoid cancer, colon cancer, thymus cancer, blood cancer, head or neck cancer or oropharyngeal cancer.

In a fifteenth aspect of the present invention, provided is use of improvement of FoxP3 expression in reducing Ryr2 expression, reducing basal Ca²⁺ oscillation, reducing m-calpain activity, or improving binding strength of a T cell to a DC cell.

In a sixteenth aspect of the present invention, provided is use of regulation of Ryr2 expression in regulating basal Ca²⁺ oscillation, regulating m-calpain activity or improving binding strength of a T cell to a DC cell. Preferably, the regulation is a reduction or an improvement.

In a seventeenth aspect of the present invention, provided is use of regulation of basal Ca²⁺ oscillation in regulating m-calpain activity or regulating binding strength of a T cell to a DC cell. Preferably, the regulation is a reduction or an improvement.

In an eighteenth aspect of the present invention, provided is use of regulation of m-calpain activity in regulating binding strength of a T cell to a DC cell. Preferably, the regulation is a reduction or an improvement.

In a nineteenth aspect of the present invention, provided is an Ryr2 antagonist, the antagonist targets exon 7 of Ryr2 gene or a guanine-rich sequence in the Ryr2 gene. Preferably, the guanine-rich sequence is located within first 200 bp from a start codon of the Ryr2 gene. More preferably, the guanine-rich sequence comprises GCAGGGG.

In a twentieth aspect of the present invention, provided is a method for regulating binding strength of a T cell to a DC cell, comprising regulating Ryr2 expression.

Preferably, the regulation is a reduction or an improvement. The method for reducing the binding strength of the T cell to the DC cell comprises improving the Ryr2 expression, or the method for improving the binding strength of the T cell to the DC cell comprises reducing the Ryr2 expression.

Preferably, the reduction of the Ryr2 expression comprises overexpressing FoxP3 in the T cell or adding an Ryr2 inhibitor. More preferably, the Ryr2 inhibitor is selected from the antagonist described herein, ryanodine, dantrolene or JTV519.

Preferably, the improvement of the Ryr2 expression comprises reducing FoxP3 expression in the T cell or adding an Ryr2 driving agent. More preferably, the Ryr2 driving agent is selected from nicotinamide adenine dinucleotide phosphate, caffeine, p-chloro-m-cresol, ryanodine, chlorantraniliprole, cyantraniliprole, beta-adrenaline, 4-chloro-3-methylphenol, cyantraniliprole, cyclaniliprole, cyclic adenosine diphosphate ribose, suramin sodium, tetraniliprole or trifluoperazine.

Preferably, the reduction of the Ryr2 expression comprises deleting exon 7 of the Ryr2 gene in the T cell.

In one specific embodiment of the present invention, the exon 7 of the Ryr2 gene is knocked out by shRNA set forth in SEQ ID NO: 5 or 6.

Preferably, the reduction of the Ryr2 expression comprises knocking down or knocking out the Ryr2 gene in the T cell. More preferably, a guanine-rich sequence of the Ryr2 gene in the T cell is knocked down or knocked out. Even more preferably, the guanine-rich sequence is located within first 200 bp from a start codon of the Ryr2 gene. Most preferably, the guanine-rich sequence comprises GCAGGGG.

Preferably, the guanine-rich sequence is a nucleotide sequence comprising at least 4 guanines (G), more preferably, a nucleotide sequence comprising at least 4-10 guanines (G), and in one specific embodiment of the present invention, comprising at least 4, 5, 6, 7, 8, 9, 10, 11, 12, 13, 14 or 15 guanines or the like.

In a twenty-first aspect of the present invention, provided is a method for reducing Ryr2 expression in a T cell, reducing basal Ca²⁺ oscillation, reducing m-calpain activity, comprising: overexpressing FoxP3 in the T cell, knocking down or out the Ryr2 gene in the T cell, or adding an Ryr2 inhibitor.

Preferably, at least exon 7 of the Ryr2 gene is knocked out.

In one specific embodiment of the present invention, the exon 7 of the Ryr2 gene is knocked out by shRNA set forth in SEQ ID NO: 5 or 6.

Preferably, the method comprises knocking down or knocking out the Ryr2 gene in the T cell. More preferably, a guanine-rich sequence of the Ryr2 gene in the T cell is knocked down or knocked out. Even more preferably, the guanine-rich sequence is located within first 200 bp from a start codon of the Ryr2 gene. Most preferably, the guanine-rich sequence comprises GCAGGGG.

Preferably, the guanine-rich sequence is a nucleotide sequence comprising at least 4 guanines (G), more preferably, a nucleotide sequence comprising at least 4-10 guanines (G), and in one specific embodiment of the present invention, comprising at least 4, 5, 6, 7, 8, 9, 10, 11, 12, 13, 14 or 15 guanines or the like.

Preferably, the Ryr2 inhibitor is selected from the antagonist described herein, ryanodine, dantrolene or JTV519.

In a twenty-second aspect of the present invention, provided is a method for improving Ryr2 expression in a T cell, improving basal Ca²⁺ oscillation, improving m-calpain activity, comprising: reducing FoxP3 expression in the T cell, or adding an Ryr2 driving agent.

Preferably, the Ryr2 driving agent is selected from nicotinamide adenine dinucleotide phosphate, caffeine, p-chloro-m-cresol, ryanodine, chlorantraniliprole, cyantraniliprole, beta-adrenaline, 4-chloro-3-methylphenol, cyantraniliprole, cyclaniliprole, cyclic adenosine diphosphate ribose, suramin sodium, tetraniliprole or trifluoperazine.

In a twenty-third aspect of the present invention, provided is a method for transforming a Tconv cell to having similar function to a Treg cell, comprising overexpressing FoxP3 in the Tconv cell, or knocking down or knocking out Ryr2 gene in the Tconv cell.

Preferably, the method comprises knocking down or knocking out at least exon 7 of the Ryr2 gene.

In one specific embodiment of the present invention, the exon 7 of the Ryr2 gene is knocked out by shRNA set forth in SEQ ID NO: 5 or 6.

Preferably, the method comprises knocking down or knocking out a guanine-rich sequence of the Ryr2 gene in the Tconv cell. More preferably, the guanine-rich sequence is located within first 200 bp from a start codon of the Ryr2 gene. Even more preferably, the guanine-rich sequence comprises GCAGGGG.

In one specific embodiment of the present invention, a nucleotide sequence of 1.5 kb or 40 kb in length in the promoter region of the Ryr2 gene is knocked down or knocked out.

Preferably, the functions similar to Treg cells include, but are not limited to, binding strength to DC cells, expression level of relevant surface markers (e.g., CD25, GITR, CTLA-4, CD39, PD-1, LAG-3, TIM-3), lower Ca²⁺ levels, low basal Ca²⁺ oscillations, low CMAC digestion, reduced talin digestion or reduced immune transitions, etc.

In a twenty-fourth aspect of the present invention, provided is a method for treating an infectious disease or inflammation, comprising administering to an individual an effective amount of the T cell with Ryr2 gene deletion described herein.

In a twenty-fifth aspect of the present invention, provided is a method for treating an infectious disease or an inflammation, the method is selected from overexpressing FoxP3 in a T cell of an individual, reducing Ryr2 expression in a T cell of an individual, reducing basal Ca²⁺ oscillation in a T cell of an individual, reducing m-calpain activity in a T cell of an individual, improving binding strength of a T cell of an individual to a DC cell, or adding an Ryr2 inhibitor to an individual.

Preferably, the Ryr2 inhibitor is selected from the antagonist described herein, ryanodine, dantrolene or JTV519.

Preferably, the infectious disease is selected from a bacterial infection, a viral infection or a fungal infection, preferably selected from a viral infection, pneumonia with septic shock, peritonitis, bacteremia, sepsis or septicemia; the viral infection is selected from an acute viral infection or a chronic viral infection, and the virus is preferably selected from influenza virus, parainfluenza virus, herpes virus (e.g., HSV-1, EBV), measles virus, vesicular stomatitis virus, hepatitis B virus, hepatitis C virus, human immunodeficiency virus, lymphocytic choriomeningitis virus or human papilloma virus.

Preferably, the inflammation may be an inflammation occurring in any tissue, the tissue includes, but is not limited to, adrenal gland, adrenal medulla, anus, appendix, bladder, blood, bone, bone marrow, brain, mammary gland, cecum, central nervous system (including or excluding brain), cerebellum, cervix, colon, duodenum, endometrium, epithelial cells (e.g., renal epithelial cells), gallbladder, esophagus, glial cells, heart, ileum, jejunum, kidney, lacrimal gland, larynx, liver, lung, lymph, lymph node, lymphoblasts, maxilla, mediastinum, mesenterium, myometrium, nasopharynx, omentum, oral cavity, ovary, pancreas, parotid gland, peripheral nervous system, peritoneum, pleura, prostate, salivary gland, sigmoid colon, skin, small intestine, soft tissue, spleen, stomach, testis, thymus, thyroid, tongue, tonsil, trachea, uterus, vulva or white blood cells. More preferably, the inflammation is selected from systemic lupus erythematosus, rheumatoid arthritis, psoriatic arthritis, scleroderma, asthma, atopic dermatitis, organ-specific inflammatory diseases, allergies (e.g., allergic rhinitis), folliculitis, tonsillitis, pneumonia, hepatitis, nephritis, acne, autoimmune diseases, chronic prostatitis, glomerulonephritis, hypersensitivity reactions, colitis, inflammatory bowel diseases, pelvic inflammatory disease, reperfusion injury, transplantation rejection, vasculitis or interstitial cystitis.

In a twenty-sixth aspect of the present invention, provided is a method for treating a tumor, comprising administering to an individual an effective amount of the T cell overexpressing the Ryr2 gene described herein.

In a twenty-seventh aspect of the present invention, provided is a method for treating a tumor, the method is selected from reducing FoxP3 expression in a T cell of an individual, improving Ryr2 expression in a T cell of an individual, improving basal Ca²⁺ oscillation in a T cell of an individual, improving m-calpain activity in a T cell of an individual, reducing binding strength of a T cell of an individual to a DC cell, or adding an Ryr2 driving agent to an individual. Preferably, the Ryr2 driving agent is selected from nicotinamide adenine dinucleotide phosphate, caffeine, p-chloro-m-cresol, ryanodine, chlorantraniliprole, cyantraniliprole, beta-adrenaline, 4-chloro-3-methylphenol, cyantraniliprole, cyclaniliprole, cyclic adenosine diphosphate ribose, suramin sodium, tetraniliprole or trifluoperazine.

Preferably, the tumor may be any adverse cell proliferation (or any disease that manifests itself as adverse cell proliferation) or neoplasm, or increased predisposition or risk for adverse cell proliferation, neoplasm or a tumor. The tumor may be benign or malignant, and may also be primary or secondary (metastatic). The neoplasm may be any abnormal growth or proliferation of cells and may be located in any tissue. Examples of the tissue include adrenal gland, adrenal medulla, anus, appendix, bladder, blood, bone, bone marrow, brain, mammary gland, cecum, central nervous system (including or excluding brain), cerebellum, cervix, colon, duodenum, endometrium, epithelial cells (e.g., renal epithelial cells), gallbladder, esophagus, glial cells, heart, ileum, jejunum, kidney, lacrimal gland, larynx, liver, lung, lymph, lymph node, lymphoblasts, maxilla, mediastinum, mesenterium, myometrium, nasopharynx, omentum, oral cavity, ovary, pancreas, parotid gland, peripheral nervous system, peritoneum, pleura, prostate, salivary gland, sigmoid colon, skin, small intestine, soft tissue, spleen, stomach, testis, thymus, thyroid, tongue, tonsil, trachea, uterus, vulva or white blood cells. More preferably, the tumor is selected from prostate cancer, breast cancer, liver cancer, glioma (e.g., neuroglioma), intestinal cancer, cervical cancer, non-small cell lung cancer, lung cancer, pancreatic cancer, gastric cancer, bladder cancer, skin cancer, striated muscle cancer, tongue squamous carcinoma, nasopharyngeal cancer, ovarian cancer, placental choriocarcinoma, lymphoma (e.g., non-Hodgkin's lymphoma, Hodgkin's lymphoma or cutaneous T-cell lymphoma), leukemia, rectal adenocarcinoma, medulloblastoma, meningioma, neurofibroma (e.g., neurofibrosarcoma), ependymoma, schwannoma, astrocytoma, melanoma, mesothelioma, myeloma, chronic granulocytic leukaemia, acute myelogenous leukemia, myelodysplastic syndrome, chronic lymphocytic leukemia, epidermoid cancer, colon cancer, thymus cancer, blood cancer, head or neck cancer or oropharyngeal cancer.

As used herein, the "regulation" or "regulating"includes an improvement (improve) or a reduction (reduce). For example, the "reagent for regulating FoxP3 expression" includes a reagent for improving the FoxP3 expression or a reagent for reducing the FoxP3 expression; the "reagent for regulating Ryr2 expression" includes a reagent for improving the Ryr2 expression or a reagent for reducing the Ryr2 expression.

As used herein, the "treatment" or "treating" includes, but is not limited to, slowing, interrupting, arresting, controlling, stopping, reducing or reversing the progression or severity of a sign, symptom, disorder, condition or disease, but does not necessarily involve the complete elimination of all disease-related signs, symptoms, conditions or disorders.

As used herein, the "reagent" represents a universal reagent, a high-purity reagent, an analytical reagent, an instrumental analytical reagent, a clinical diagnostic reagent, a biochemical reagent, an inorganic ion color reagent, an apparatus, or a device, which can generate a corresponding function or be used for a certain purpose. For example, the "reagent for improving FoxP3 expression" includes any reagent that can improve the FoxP3 expression in T cells, for example, a reagent for introducing the FoxP3 gene into the T cells, a reagent for improving transcription or expression of the FoxP3 gene in the T cells, and the like. The "reagent for reducing FoxP3 expression" includes any reagent that can reduce the FoxP3 expression in T cells, for example, a reagent for knocking out or knocking down the FoxP3 gene in the T cells, a reagent for inhibiting transcription or expression of the FoxP3 gene, and the like. The "reagent for reducing Ryr2 expression" includes, but is not limited to, a desired reagent for knocking down or knocking out the Ryr2 gene, or a reagent for improving the FoxP3 expression, or a reagent for reducing transcription or expression of the Ryr2, such as the antagonist described herein, ryanodine, dantrolene or JTV519, and the like. The "reagent for improving Ryr2 expression" includes, but is not limited to, an agent for introducing Ryr2 gene into T cells, an agent for increasing Ryr2 gene transcription or expression in T cells, or an agent for reducing FoxP3 expression in T cells, and the like. The "reagent for reducing basal Ca²⁺ oscillation" includes, but is not limited to, a reagent for reducing the Ryr2 expression. The "reagent for improving basal Ca²⁺ oscillation" includes, but is not limited to, a reagent for improving the Ryr2 expression. The "reagent for reducing m-calpain activity" includes, but is not limited to, a reagent for reducing the Ryr2 expression. The "reagent for improving m-calpain activity" includes, but is not limited to, a reagent for improving the Ryr2 expression. The "reagent for improving binding strength of a T cell to a DC cell" includes, but is not limited to, a reagent for reducing the Ryr2 expression. The "reagent for reducing binding strength of a T cell to a DC cell" includes, but is not limited to, a reagent for improving the Ryr2 expression.

As used herein, the "individual" includes, but is not limited to, a non-human mammal or a human. Preferably, the non-human mammal includes, but is not limited to, a monkey, a dog, a mouse or a rat, etc.

As used herein, the "effective amount" is an amount or dose of the reagent or medicament described herein which provides the desired treatment after being administered to an individual or organ in a single dose or multiple doses.

As used herein, the "CMAC" is a blue fluorescent dye with a chemical name of 7-amino-4-chloromethylcoumarin.

### BRIEF DESCRIPTION OF THE DRAWINGS

Hereinafter, embodiments of the present invention will be described in detail with reference to the accompanying drawings, in which:
FIG. 1 shows protein expression analysis of m-calpain in Tconv and Treg cells isolated from wild-type mice; wherein, FIGs. A and C shows the mRNA level of m-calpain, and FIG. 1B shows the protein level of m-calpain; all values are expressed in mean value + SEM.
FIG. 2 shows basal Ca²⁺ oscillations in single Tconv and Treg cells in the resting state, detected in an HBSS system containing 2 mM Ca²⁺ with addition of Ca²⁺ probe Fluo 4-AM; wherein each line represents one cell, n represents the number of biological replicates in a set of data points, and n = 20.
FIG. 3 shows mean fluorescence intensities (MFIs) of Ca²⁺ oscillations in Tconv and Treg cells in the resting state. The Tconv and Treg cells were stained with Fluo 4-AM, and analyzed for the MFIs.
FIG. 4 shows standard deviations (SDs) of Ca²⁺ oscillation intensities shown in FIG. 2.
FIG. 5 shows the levels of calcium ion regulation-related proteins between Treg and Tconv cells from GEO database analysis sorted by the difference.
FIG. 6 shows the Ryr2 mRNA expression in purified Tconv and Treg cells by qPCR analysis.
FIG. 7 shows the determination of calpain activity in Tconv (left) and Treg (right) cells after Ryr activation (4-CMC treatment) by digestion of the calpain substrate CMAC.
FIG. 8 shows basal calcium ion oscillations in Tconv cells with Ryr2 inhibited using 5 mM JTV519 inhibitor (middle), wild-type Tconv cells (left) and Treg cells (right).
FIG. 9 shows standard deviations of basal calcium ion oscillations in Tconv cells with Ryr2 inhibited using 5 mM JTV519 inhibitor, wild-type Tconv cells and Treg cells.
FIG. 10 shows transcription levels of mRNA in Tconv cells isolated from WT mice and Tconv cells with Ryr2 gene knock down using shRNA, analyzed by qPCR.
FIG. 11 shows basal Ca²⁺ oscillations in Tconv cells with Ryr2 gene knock down using shRNA vs. wild-type Tconv cells.
FIG. 12 shows standard deviations of basal Ca²⁺ oscillations in Tconv cells with Ryr2 gene knock down using shRNA vs. wild-type Tconv cells.
FIG. 13 shows calpain activities in Tconv after JTV519 treatment (left), Treg after JTV519 treatment (middle) and Tconv with Ryr2 knock down (right).
FIG. 14 shows the detection of adhesion of T cells to DC2.4, wherein, the left panel shows the mean adhesion of untreated Treg and Tconv and JTV519-treated Tconv, and the right panel shows the mean adhesion of blank interference control (shCtrl) and Tconv with Ryr2 knock down.
FIG. 15 shows the adhesion assay between OT-II T cells and OVA-presenting DC2.4 cells, wherein FIG. A shows the triplecell AFM analysis pattern, and FIG. B shows the mean adhesion of W/O cells (cell-free group), blank interference control (shCtrl), and Tconv with Ryr2 knock down and Treg as suppressor cells.
FIG. 16 shows test results of inhibiting the division of OT-II T cells, wherein FIG. A is the FACS patterns of inhibiting the division of OT-II T cells by Treg-free, Treg, blank interference control (shCtrl) or Tconv cells with Ryr2 knock down, and FIG. B shows the inhibitory efficiency of either blank interference control (shCtrl) or Tconv cells with Ryr2 knock down normalized by the efficiencies of Treg (defined as 100% inhibition) and no Treg (defined as 0% inhibition), wherein, * represents P < 0.05, ** represents p < 0.01, *** represents p < 0.001, and **** represents p < 0.0001.
FIG. 17 shows shRNA knock down efficiency of Itpr1, Cacnb1, Trpm1, Trpm4, Trpv2, Orai1 and Orai3 genes in Tconv cells isolated from WT mice analyzed using qPCR.
FIG. 18 shows the mean adhesion in untreated Treg cells, blank interference control Tconv (shCtrl), Tconv with Ryr2 knock down, Tconv with Itpr1 knock down, Tconv with Cacnb1 knock down, Tconv with Trpm1 knock down, Tconv with Trpm4 knock down, Tconv with Trpv2 knock down, Tconv with Orai1 knock down, and Tconv with Orai3 knock down.
FIG. 19 shows that FoxP3 overexpression silences transcription of Ryr2 gene, and specifically, shows Ryr2 mRNA levels in Tconv, A20, 3T3 and Renca cells with FoxP3 overexpression.
FIG. 20 shows that FoxP3 overexpression silences transcription of Ryr2 gene. In the Foxp3 ChIP-seq, shown is Tconv with Foxp3 transfection (upper) or Tconv without Foxp3 transfection (lower). The TSS site of the Ryr2 gene is indicated by an arrow. The triangle denotes significantly different binding peak identified by ChIP-seq, the position of which mainly overlaps with the promoter region (box) of the gene.
FIG. 21 shows that FoxP3 overexpression silences transcription of Ryr2 gene. The upper panel shows the construction of Ryr2 promoter-luciferase reporter vector, and TSS represents the transcription start site. The lower panel shows the transcription level of driven by the Ryr2 promoter in 3T3 or Renca cells with FoxP3 overexpression.
FIG. 22 shows that FoxP3 overexpression silences transcription of Ryr2 gene. FIG. A is a schematic diagram of truncated vector of the Ryr2 promoter-luciferase reporter vector, showing the result of sequential segment deletion performed in the sequence, and FIG. B shows the transcription level driven by the Ryr2 promoter in 3T3 cells with FoxP3 overexpression.
FIG. 23 shows that FoxP3 overexpression silences transcription of Ryr2 gene, wherein FIG. A is a schematic diagram of 2 truncated vectors and knock out strategy of Ryr2 promoter-luciferase reporter vector, and FIG. B shows the transcription levels driven by the Ryr2 promoter expressed by different truncated vectors or FoxP3 binding sequence in 3T3 cells with FoxP3 overexpression, wherein N.S. represents no significance.
FIG. 24 shows that FoxP3 overexpression silences transcription of Ryr2 gene. In HBSS without calcium ions, 4-CmC stimulated A20 or 3T3 cells with FoxP3 overexpression, which is illustrated by the mean trajectory of calcium ions over time, wherein N = 3, and at least 30 cells were collected for analysis in a single measurement in each independent experiment.
FIG. 25 shows that T cells (Ryr2-/- Tconv) with Ryr2 gene knock down induced immunosuppression in mouse psoriasis (Scurfy) model and restored immune homeostasis in Scurfy mice, wherein FIG. A shows the survival rates of WT mice and mice receiving PBS injection, and FIG. B shows the survival rates of mice receiving Treg, Ryr2+/+(FoxP3-) Tconv or Ryr2-/- Tconv injection.
FIG. 26 shows the conditional knock down mouse construction of Cre-dependent knock down exon 7 of Ryr2 gene.
FIG. 27 shows the distribution of CD4+ and CD8+ T cells in the spleen and thymus of Ryr2+/+ mice (wild-type mice without Ryr2 knock out), CD4-Cre/Ryr2f1/+ mice, and CD4-Cre/Ryr2-/- mice by flow cytometry.
FIG. 28 shows the proportion of FoxP3+/CD4+ spleen cells in mice.
FIG. 29 shows the flow cytometry analysis of surface makers related to Treg functions (including CD25, GITR, CTLA-4, CD39, PD-1, LAG-3, TIM-3) in WT Tconv (Ryr2+/+) and Ryr2-/- Tconv, and ELISA of IL-10 and TGF- β levels in the supernatants after stimulation with anti-CD3/anti-CD28 for 24 h.
FIG. 30 shows that T cells with Ryr2 knock down have inhibitory function *in vitro* based on the cell contact, and shows the calcium ion oscillation levels and their standard deviation in HBSS containing 2 mM Ca²⁺ (FIG. D) by the comparison of Tconv isolated from WT (FIG. A) or CD4-Cre, Ryr2fl/fl (Ryr2-/-) (FIG. B) mice with Treg (FIG. C).
FIG. 31 shows the detection of calpain activity in Treg, Ryr2+/+ and Ryr2-/- Tconv cells by CMAC substrate cleavage.
FIG. 32 shows the results of substrate Talin cleavage *in vivo* in triple cell lines, Treg, Ryr2+/+ and Ryr2-/- Tconv, by western blotting.
FIG. 33 shows that Tconv cells with Ryr2 knock down have inhibitory function *in vitro* based on the cell contact; all data points (points in FIGs. A and B) from four independent DC-T cell pairs in each condition collected on the same day were used to generate the bar graph of FIG. C, with at least 15 adhesion readings collected for each T-DC pair; specifically, shown are the adhesion of WT Tconv (FIG. A) and Ryr2-/- Tconv cells (FIG. B) to DC2.4, and the mean adhesion (FIG. C).
FIG. 34 shows the adhesion experiment between OT-II T cells and OVA-presenting DC2.4 cells, and the suppressor cells on the DC are Treg, WT Tconv or Ryr2-/- Tconv cells; wherein, FIG. A shows a triple-cell AFM measuring device, and FIG. B shows the mean adhesion of OT-II-DC in Treg, WT Tconv or Ryr2-/- Tconv as suppressor cells.
FIG. 35 shows the relative inhibitory efficiency without Treg, or with Treg, Ryr+/+ and Ryr2-/- Tconv, wherein the relative inhibitory efficiency in Treg-free group is defined as 0%, and the relative inhibition efficiency in Treg group is defined as 100%.
FIG. 36 shows that T cells (Ryr2-/- Tconv) with knock down of Ryr2 gene induced immunosuppression in a mouse model with herpes infection. In the figure, shown is analysis of HSV-1 titers in footpad tissues after cell adoptive transfer, wherein HSV-1, Treg, Ryr2-/- Tconv or Ryr2+/+ Tconv denote adoptive transfer PBS, Treg or Ryr2-/- Tconv, Ryr2+/+ Tconv, respectively.
FIG. 37 shows that T cells (Ryr2-/- Tconv) with Ryr2 gene knock down induced immunosuppression in a mouse model with herpes infection. In the figure, shown is delayed-type hypersensitivity (DTH) after cell adoptive transfer and re-challenge with UV-inactivated HSV-1 antigen, wherein the footpad thickness without antigen re-challenge is set at 100%.
FIG. 38 shows that T cells (Ryr2-/- Tconv) with Ryr2 gene knock down induced immunosuppression in a mouse model with herpes infection, wherein the upper panel shows the protocol in the DTH assay and the lower panel shows the representative footpad swelling.
FIG. 39 shows that T cells (Ryr2-/- Tconv) with Ryr2 gene knock down induced immunosuppression in a mouse model with asthma, wherein FIG. A shows the total infiltration of BALF, FIG. B shows the infiltration of lymphocytes, and FIG. C shows the infiltration of eosinophils. The values of the assay results are the mean values in 3 trials, the blank represents blank control group, and PBS, Treg, Ryr2-/- Tconv and Ryr2+/+ Tconv groups are asthma models with injection of an equal amount of PBS, Treg, Ryr2-/- Tconv and Ryr2+/+ Tconv.
FIG. 40 shows that T cells (Ryr2-/- Tconv) with Ryr2 gene knock down induced immunosuppression in a mouse model with asthma, wherein FIG. A shows the sensitization scheme of the asthma model, FIG. B shows a representative lung H&E tissue section, and the scale bar represents 200 µm. PBS, Treg, Ryr2-/- Tconv and Ryr2+/+ Tconv groups were asthma models receiving an equal amount of PBS, Treg, Ryr2-/- Tconv and Ryr2+/+ Tconv injection.
FIG. 41 shows that T cells (Ryr2-/- Tconv) with Ryr2 gene knock down induced immunosuppression in a mouse model with inflammatory bowel disease (IBD), wherein the blank represents blank control, and DSS represents water treatment.
FIG. 42 shows that T cells (Ryr2-/- Tconv) with Ryr2 gene knock down induced immunosuppression in a mouse model with inflammatory bowel disease (IBD), wherein FIG. A shows the induction protocol of the DSS-induced colitis model, FIG. B shows the representative colon image, FIG. C shows the micrograph of a colon section, and the scale bar represents 200 µm.
FIG. 43 shows that T cells (Ryr2-/- Tconv) with Ryr2 gene knock down induced immunosuppression in a mouse model with colon cancer, and tumor volume growth curves are measured separately for each group of mice, group 1: MC38 + PBS; group 2: MC38 + Ryr2+/+ Tconv; group 3: MC38 + Treg; and group 4: MC38 + Ryr2-/- Tconv.
FIG. 44 shows that T cells (Ryr2-/- Tconv) with Ryr2 gene knock down induced immunosuppression in mouse psoriasis (Scurfy) model and restore immune homeostasis in Scurfy mice. In the figure, shown are Kaplan-Meier survival curves in mice, wherein FIG. A shows the survival rates of WT mice and mice receiving PBS injection, and FIG. B shows the survival rates of mice receiving Treg, Ryr2+/+(FoxP3-) Tconv or Ryr2-/- Tconv injection.
FIG. 45 shows that T cells (Ryr2-/- Tconv) with Ryr2 gene knock down induced immunosuppression in mouse psoriasis (Scurfy) model and restore immune homeostasis in Scurfy mice, wherein FIG. A shows the physical symptoms and treatment in Scurfy mice, and
FIG. B shows the representative H&E staining of each organ (thymus, spleen, lung, ear, liver, pancreas, small intestine and colon) in model mice injected with PBS and WT mice. Samples were taken at week 3, and mice adoptively transferred Treg cells, Foxp3- (Ryr2+/+) Tconv and Ryr2-/- Tconv were taken at weeks 8-12.

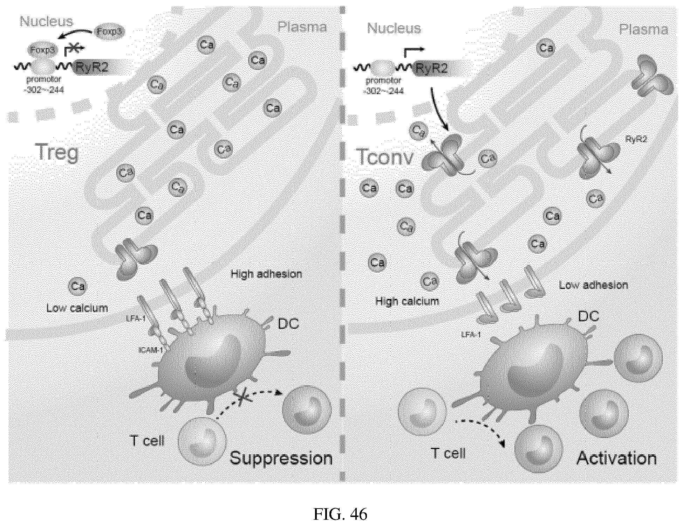

FIG. 46 shows that overexpressing Foxp3 to inhibit Ryr2 is a key effect mechanism of T cell immunosuppression, wherein the left panel shows the action mechanism in T cells after Foxp3 overexpression inhibited Ryr2, and the right panel shows the action mechanism in wild-type T cells.

### DETAILED DESCRIPTION OF THE EMBODIMENTS

Technical schemes in the examples of the present invention will be described clearly and completely in conjunction with the accompanying drawings. It is apparent that the examples described herein are only some examples of the present invention, but not all of them. Based on the examples of the present invention, all other examples obtained by those of ordinary skill in the art without intensive steps shall fall within the protection scope of the present invention.

Sources of test animals, reagents and devices used in the examples are as follows.

Mice: All mice were in C57BL/6 background. Ryr2fl/fl mice and CAG-LSL-Ryr2-flag transgenic mice were produced by GemPharmatech Co., Ltd, and identified by genotype. OT-II transgenic mice were purchased from Jackson laboratory. Wild-type mice, Foxp3-IRES-GFP mice, Ryr2fl/fl mice, Ryr2 transgenic mice, OT-II transgenic mice, CD4-Cre mice and Foxp3-Cre-YFP mice, female Foxp3+/- mice were bred and housed at Tsinghua University Animal Facilities. All mice were bred in specific pathogen-free (SPF) conditions. All animal experiments met animal welfare guidelines and were approved by the Institutional Animal Care and Use Committee (IACUC) of Tsinghua University.

Cell lines and primary cells culture: DC2.4 cells were provided by Dr. Ken Rock in UMass Medical School; Vero cells were provided by Dr. Tan Xu in School of Pharmaceutical Science of Tsinghua university; Renca cells were provided by doctor An Guangyu in Beijing Chao-Yang Hospital; HEK293FT cells were provided by Dr. Guo Wei in School of Medicine of Tsinghua University; and MC38 cells were purchased from American Type Culture Collection (ATCC). MC38, Renca, NIH-3T3 cells and Vero cells were cultured in DMEM containing 10% FBS, 100 U/mL penicillin and 100 mg/mL streptomycin. All other cells were grown in RPMI-1640 at the same dose containing 50 µM β-mercaptoethanol. All cell lines were tested for mycoplasma contamination by PCR analysis. Mouse CD4+ CD25+ Treg and CD4+ CD25- Tconv were isolated from the spleen using a mouse CD4+ T cell isolation kit (StemCell, 19852) and a mouse CD25 Treg cell positive selection kit (Stem cell, 18782). Treg and Tconv cells were sometimes selected by FACS from Foxp3-IERS-GFP mouse CD4+ spleen cells. Mouse DCs were isolated from the spleen using mouse CD11c selection kit II (Stem cell, 28007). OT-IIT cells were isolated from OT-II spleen cells using mouse CD4+ T cell isolation kit (Stem cell, 19852), and sometimes sorted by FACS with anti-TCRVα2 antibodies.

Antibodies and reagents: All primary cell isolation kits were from StemCell. Recombinant human IL-2 was from R&D systems. Anti-mouse CD3e monoclonal antibody, and anti-mouse CD28 and anti-mouse TCRVα2-PE antibodies were from eBioscience. Flow antibodies were from eBioscience, including anti-CD4, anti-CD8, anti-GITR, anti-CD25, anti-PD-1, anti-CTLA-4, anti-Tim-3 and anti-LAG3 antibodies. Anti-FoxP3 antibodies were from Invitrogen. The following clones were used: anti-mouse CD4, anti-mouse CD8, anti-mouse GITR, anti-mouse CD25, anti-mouse PD-1, anti-mouse/rat CTLA-4, anti-mouse/rat FoxP3 (FJK-16s), anti-mouse/human Tim-3, anti-mouse LAG3, anti-mouse PD-1 (J43.1), anti-mouse CTLA4 (UC10-4B9) and anti-mouse GITR (DTA-1). The following antibodies were used for western blotting: m-calpain large subunit (M-type) antibodies and anti-mouse IgG-HRP linked antibodies from CST. Anti-talin antibodies (8D4) were purchased from Sigma-Aldrich.
pLKO.1 vectors purchased from shRNA library platform in the Center of Biomedical Analysis of Tsinghua University were used for all our gene knock down studies. All pre-designed shRNA sequences were synthesized by Ruiboxingke Biotech.
pLVX-IRES-mcherry vectors were provided by Dr. ShenXiaohua in School of Medicine of Tsinghua University.

### Example 1: Preparation of T cells with Ryr2 gene knock down or FoxP3 overexpression

### 1. Procedures for knocking down Ryr2 gene

Lentivirus-based shRNA was used to knock down Ryr2 gene, and specifically, pre-synthesized shRNA sequences were inserted into pLKO.1 vectors. Plasmids including shRNA and packaging constructs (pMD2.G and psPAX2) were purified from transformed *Escherichia coli* using the EndoFree plasmid Midi kit (CWBIO, CW2105S). Lentiviral packaging was performed according to the manufacturer's instructions. Briefly, 293FT cells were cultured in 10 cm dishes to a 60% to 80% coverage. The media were replaced 2 h before DNA transfection. 2.5 µg of packaging vectors pMD2.G and 2.5 µg of psPAX2, and 5 µg of pLKO. 1 vectors (with inserted shRNA) were transfected into 293FT cells using a Neofect system (Neofect). After 72 h, lentiviruses were harvested and T cells were infected with Polybrene (final concentration of 8 µg/mL). Cells were sorted by Aria cytometer (BD Biosciences) 48 h after viral infection. The knock down efficiency was verified by real-time quantitative PCR. The shRNA sequences are as follows:
control-shRNA: 5'-CCGGcaacaagatgaagagcaccaaCTCGAGttggtgctcttcatcttgttgTTTTTG-3' (SEQ ID NO: 3) and 5'-AATTCAAAAAcaacaagatgaagagcaccaaCTCGAGttggtgctcttcatcttgttgg-3' (SEQ ID NO: 4); Ryr2-shRNA: 5'-CCGGccgctaatgaagccatataaaCTCGAGTTTATATGGCTTCATTAGCGGTTTTTG-3' (SEQ ID NO: 5) and 5'-AATTCAAAAAccgctaatgaagccatataaaCTCGAGTTTATATGGCTTCATTAGCGG-3' (SEQ ID NO: 6); Itpr1-shRNA: 5'-CCGGgcagtaggtaagaagttattaCTCGAGtaataacttcttacctactgcTTTTTG-3' (SEQ ID NO: 7) and 5'-AATTCAAAAAgcagtaggtaagaagttattaCTCGAGtaataacttcttacctactgc-3' (SEQ ID NO: 8);
Orai1-shRNA: 5'-CCGGcacaaccaccaactcggtcaaaCTCGAGtttgaccgagttgaggttgtgTTTTTG-3' (SEQ ID NO: 9) and 5'-AATTCAAAAAcacaacctcaactcggtcaaaCTCGAGtttgaccgagttgaggttgtg-3' (SEQ ID NO: 10); Orai3-shRNA: 5'-CCGGgcccttgcttatctgtataatCTCGAGattatacagataagcaagggcTTTTTTGG-3' (SEQ ID NO: 11) and 5'-AATTCAAAAAAgcccttgcttatctgtataatCTCGAGattatacagataagcaagggc-3' (SEQ ID NO: 12); Trpm1-shRNA: 5'-CCGGcggagtgaacatgcagcatttCTCGAGaaatgctgcatgttcactccgTTTTTG-3' (SEQ ID NO: 13) and 5'-AATTCAAAAAcggagtgaacatgcagcatttCTCGAGaaatgctgcatgttcactccg-3' (SEQ ID NO: 14); Trpm4-shRNA: 5'-CCGGgcacatcttcacggtgaacaaCTCGAGttgttcaccgtgaagatgtggttTTTG-3' (SEQ ID NO: 15) and 5'-AATTCAAAAAgcacatcttcacggtgaacaaCTCGAGttgttcaccgtgaagatgtgc-3' (SEQ ID NO: 16); Trpv2-shRNA: 5'-CCGGccaaggaacttgtttctatttCTCGAGaaatagaaacaagttccttggTTTTTG-3' (SEQ ID NO: 17) and 5'-AATTCAAAAAccaaggaacttgtttctatttCTCGAGaaatagaaacaaagagctccttgg-3' (SEQ ID NO: 18); Cacnb1-shRNA: 5'-CCGGtaggaacgcaatggatattaaCTCGAGttaatatccattgcgttcctaTTTTTG-3' (SEQ ID NO: 19) and 5'-AATTCAAAAAtaggaacgcaatggatattaaCTCGAGttaatattccattgcgttccta-3'(SEQ ID NO: 20).

### 2. Knock down efficiency verification by real-time quantitative PCR

The specific procedures are as follows:
Total RNA was extracted from the indicated cells using TRIzol reagent (Invitrogen), and a first strand cDNA was synthesized using reverse transcriptase M-MLV (TaKaRa). Real-time quantitative PCR was performed using SYBR Green Master Mix (No Rox) (Yeasen). GAPDH or 18S RNA was used for normalization control. The primer sequences were as follows:
mouse GAPDH: 5'-CATCACTGCCACCCAGAAGACTG-3' (SEQ ID NO: 21) and 5'-ATGCCAGTGAGCTTCCCGTTCAG-3' (SEQ ID NO: 22); mouse 18S RNA: 5'-CGGACAGGATTGACAGATTG-3' (SEQ ID NO: 23) and 5'-CGGACAGGATTGACAGATTG-3' (SEQ ID NO: 24); mouse Ryr2: 5'-ATGGCTTTAAGGCACAGCG-3' (SEQ ID NO: 25) and 5'-CAGAGCCCGAATCATCCAGC-3' (SEQ ID NO: 26); mouse FoxP3: 5'-CCCATCCCCAGGAGTCTTG-3' (SEQ ID NO: 27) and 5'-ACCATGACTAGGGGCACTGTA-3' (SEQ ID NO: 28); mouse m-calpain: 5'-3' and 5'-3'; mouse Itpr1: 5'-CGTTTTGAGTTTGAAGGCGTTT-3' (SEQ ID NO: 29) and 5'-CATCTTGCGCCAATTCCCG-3' (SEQ ID NO: 30); mouse Orai1: 5'-GATCGGCCAGAGTTACTCCG-3' (SEQ ID NO: 31) and 5'-TGGGTAGTCATGGTCTGTGTC-3' (SEQ ID NO: 32); mouse Orai3: 5'-GGCTACCTGGACCTTATGGG-3' (SEQ ID NO: 33) and 5'-GCAGGCACTAAATGTGACC-3' (SEQ ID NO: 34); mouse Trpm1: 5'-ATCCGAGTCTCCTACGACACC-3' (SEQ ID NO: 35) and 5'-CAGTTTGGACTGCATCTCGAA-3' (SEQ ID NO: 36); mouse Trpm4: 5'-GGACTGCACACAGGCATTG-3' (SEQ ID NO: 37) and 5'-GTACCTTGCGGGGAATGAGC-3' (SEQ ID NO: 38); mouse Trpv2: 5'-TGCTGAGGTGAACAAAGGAAAG-3' (SEQ ID NO: 39) and 5'-TCAAACCGATTTGGGTCCTGT-3' (SEQ ID NO: 40); mouse Cacnb1: 5'-GGCAGCAAGTTATCTCCCAG-3' (SEQ ID NO: 41) and 5'-CCACAGGATGATTGGCGTCTT-3' (SEQ ID NO: 42); HSV-1 gB: 5'-AACGCGACGCACATCAAG-3' (SEQ ID NO: 43) and 5'-CTGGTACGCGATCAGAAAGC-3' (SEQ ID NO: 44); HSV-1 LAT: 5'-GGGTGGGCTCGTGTTACAG-3' (SEQ ID NO: 45) and 5'-GGACGGGTAAGTAACAGAGTCTCTA-3' (SEQ ID NO: 46).

### 3. Procedures for overexpressing FoxP3

Vectors were constructed, cDNA of Treg total RNA was used as template, and FoxP3 was amplified by PCR using the following primers: the forward primer 5'-ATCGCTCGAGATGTGCACACCTAGGCCA-3' (SEQ ID NO: 47) and the reverse primer 5'-ATCGGAATTCTCAAGGGCAGGGATTGGA-3' (SEQ ID NO: 48). The amplified fragments were purified by a gel, digested (XhoI and EcoRI) and cloned into pLVX-IRES-mcherry plasmids to give a pLVX-FoxP3-IRES-mcherry construct. Lentivirus production in cell lines carrying pLVX-FoxP3-IRES-mcherry and FoxP3-overexpression was performed according to the knock down protocol described above. The FoxP3 expression was verified by RT-PCR.

### 4. Procedures of flow cytometry

The cells were incubated with Fc blocking agent (CD16/32 antibodies; 2.4G2) for 5 min, and then incubated with surface antibodies (CD4, CD8, GITR, CD25, PD-1, CTLA-4, TIM-3, LAG3) for 15 min at room temperature away from the light. FoxP3 cells were stained, and surface-stained cells were incubated with FoxP3/transcription factor immobilization/permeation buffer (Thermo Fisher) for 30 min, followed by incubation with antibodies for 2 h. Stained cells were directly analyzed, or occasionally analyzed after immobilization with 1% paraformaldehyde (PFA) using FACS Diva software and Fortessa cytometer (BD Biosciences). Data was analyzed by Flowjo software.

### 5. Procedures of ELISA

In order to detect IL-10 and TGF-β, 10⁶ purified Treg, Ryr2+/+ or Ryr2-/- Tconv cells were stimulated with anti-CD3/anti-CD28 antibodies. After 72 h, the supernatant was collected. High-binding 96-well ELISA plates (Nunc) were coated with anti-mouse IL-10 and anti-mouse TGF-β capture antibodies and incubated overnight at 4 °C. After drying, the plates were blocked with 2% BSA in PBS for 1 h at room temperature. After washing, 100 µL of diluted cell supernatant was added to triplicate wells and incubated for 1 h at room temperature. The plates were then washed with PBST (0.05% Tween20, Sigma-Aldrich, in PBS) and incubated with HRP-labeled goat anti-mouse IL-10 and TGF-β detection antibodies for 0.5 h at room temperature. TMB (100 mL/well) was added and the plates were incubated at room temperature for 10 min in the dark, and then H₂SO₄ (50 µL, 1 M) was added per well to stop the reaction. The optical density (OD) was immediately read at 450 nm using an ELISA microplate reader (Bio-Rad).

### 6. Calcium imaging

For non-adherent cells, Treg, Tconv and A20 cells were stained with 2 mM fluo-4 AM (Thermo Fisher) in HBSS-HEPS (10 mM) buffer for 1 h at 37 °C. After washing, the cells were adhered to poly-L-lysine coated (0.1 mg/mL; Sigma-Aldrich) round glass slides mounted in the sandwich home-made chamber at room temperature. After 15 min, excess non-adherent cells were removed by washing with buffer. For adherent cells, NIH-3T3 and Renca cells adherent to glass slides were stained with 2 mM fluo-4 AM (Thermo Fisher) in HBSS-HEPS (10 mM) buffer for 1 hour at 37 °C. The measurement chamber was then placed on an Olympus IX-73 microscope equipped with a 20-fold (numerical aperture: 0.8) or 40-fold (numerical aperture: 1.2) Olympus objective. Unless otherwise stated, Ca²⁺ oscillation within 20 min was recorded at intervals of 6 s. In the inhibition experiments, Fluo-4-labeled cells were treated with JTV519 (Sigma) at room temperature in the dark for 30 min, and then images were acquired. For the stimulation experiments, Ca²⁺ oscillation within 5 min was recorded at intervals of 1 s. 50 to 80 s after starting to acquire images, 4-CmC (Sigma) buffer was added to induce the release of intracellular calcium. The experiments were performed in phenol red-free HBSS medium with or without Ca²⁺. The emission signal at 468 nm to 550 nm excited by the 488 nm laser was recorded with a charge coupled device camera (ORCA-AG, Hamamatsu). Data collection was controlled by NIS-Elements 3.0 software (Nikon). The mean fluorescence intensity changes over time in individual cells were analyzed using ImageJ and normalized with fluorescence intensity in the first frame (Fluo-4 F / F0). The peak of Ca²⁺ oscillation of the individual cells was shown, and the standard deviation of Ca²⁺ fluctuation intensity was calculated as Mean ± SD.

### 7. Procedure of western blot

Cells were collected, and lysed with RIPA buffer (Beyotime, P0013B). The cell lysate was centrifuged and the supernatant was collected. Total protein was quantified using BCA protein assay kit (Beyotime, P0012). After mixing with 3XSDS loading buffer and being boiled for 5 min, the proteins were loaded onto 7.5% PAGE gels (EpiZyme, PG111). The proteins were then transferred to NC membranes, and immunoblotted with primary and secondary antibodies. Finally, immunostained bands were detected using Super ECL detection reagent (Yeasen, 36208ES76).

### 8. Results

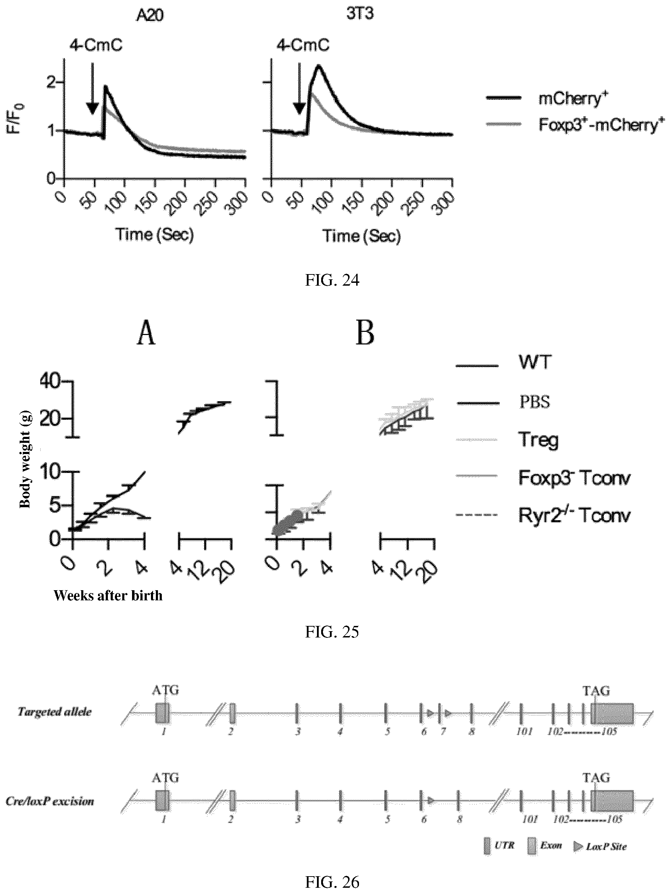

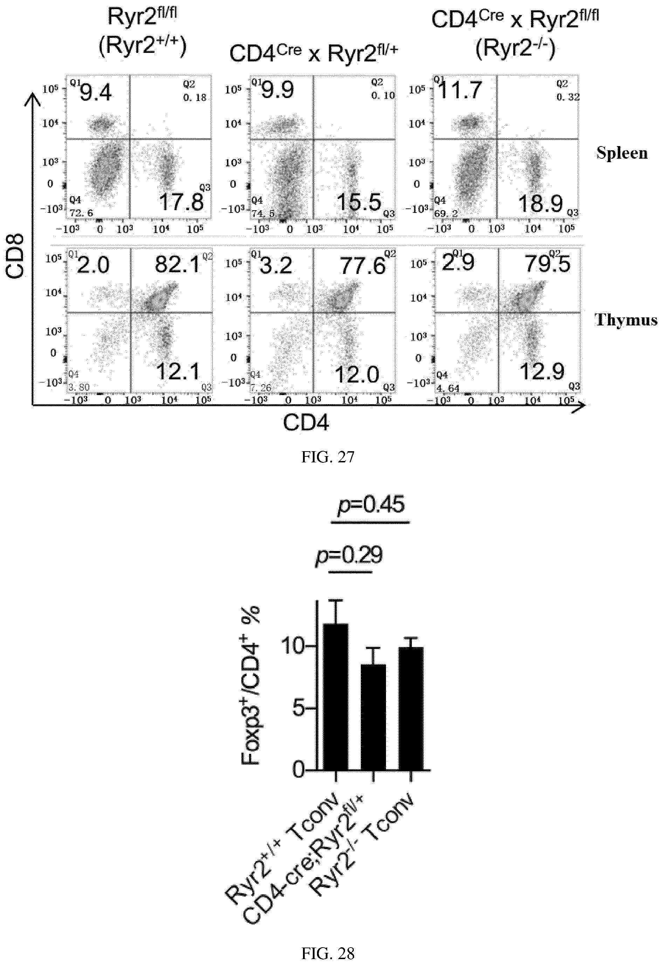

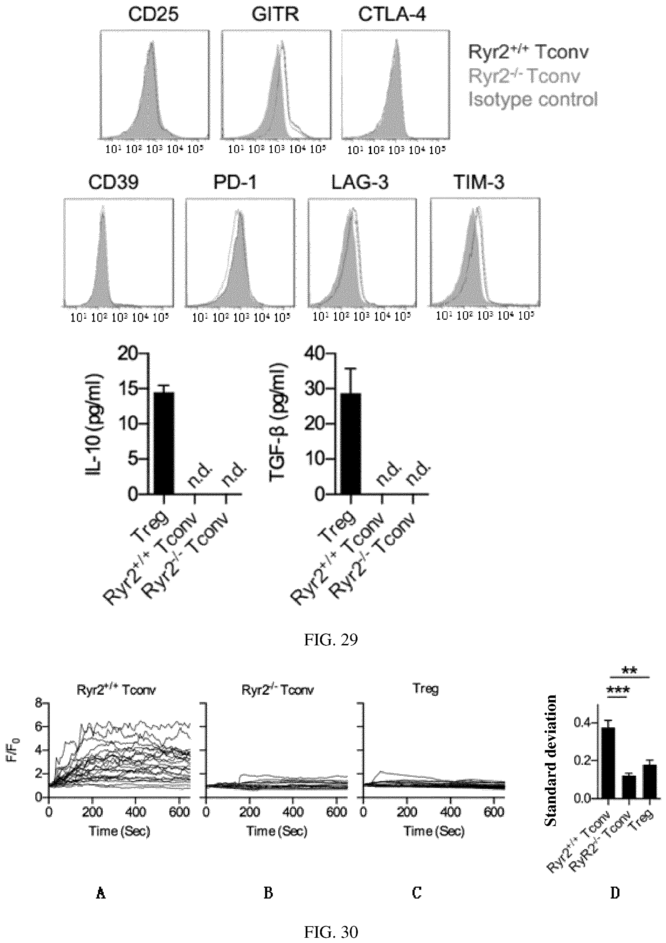

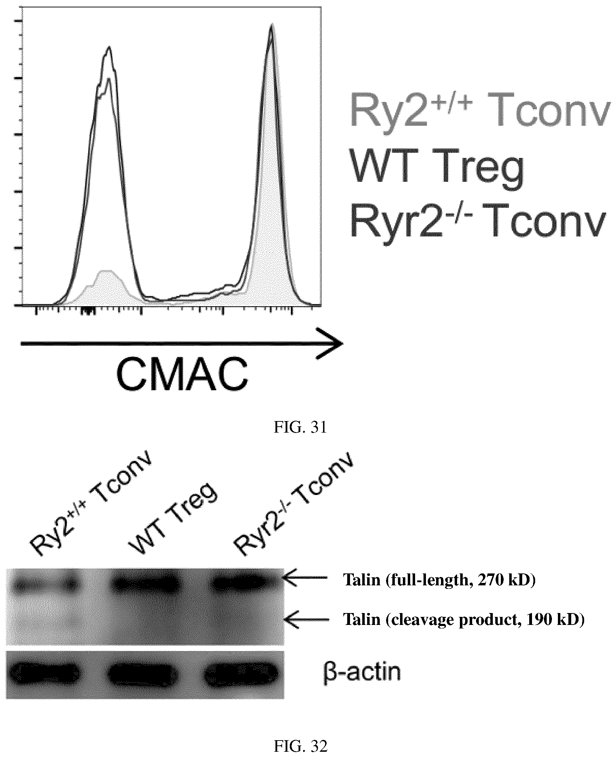

Ryr2 could be knocked down in CD4-cre Tconv (FIG. 26). Meanwhile, basic functions in the Tconv cells with knock down of Ryr2 were not affected, including the body weight and development speed of mice. The distribution of thymocytes and peripheral blood CD4+, CD8+ markers were almost the same as that in WT mice (flow assay results are shown in FIG. 27). The proportion of FoxP3+CD4+ T cells also remained unchanged (FIG. 28). Surface markers likely related to Treg functions also remained unchanged, as compared to WT Tconv (flow and ELISA assay results are shown in FIG. 29). Tconv with Ryr2 knock down was accompanied by a reduction in basal Ca²⁺ level and a reduction in oscillation intensity (calcium imaging results are shown in FIG. 30). CMAC digestion in Ryr2-/- Tconv was reduced to a level similar to Treg, as compared to untreated Tconv (FIG. 31). As with Treg, The talin digestion was largely absent in Ryr2-/- Tconv (detection results of western blot shown in FIG. 32).

### Example 2: Verification of effect of FoxP3 expression on Ryr2 gene and determination of target sites

In order to determine the specificity of Ryr2 gene regulation, in this case, it will be linked with FoxP3 expression and determined the target sites.

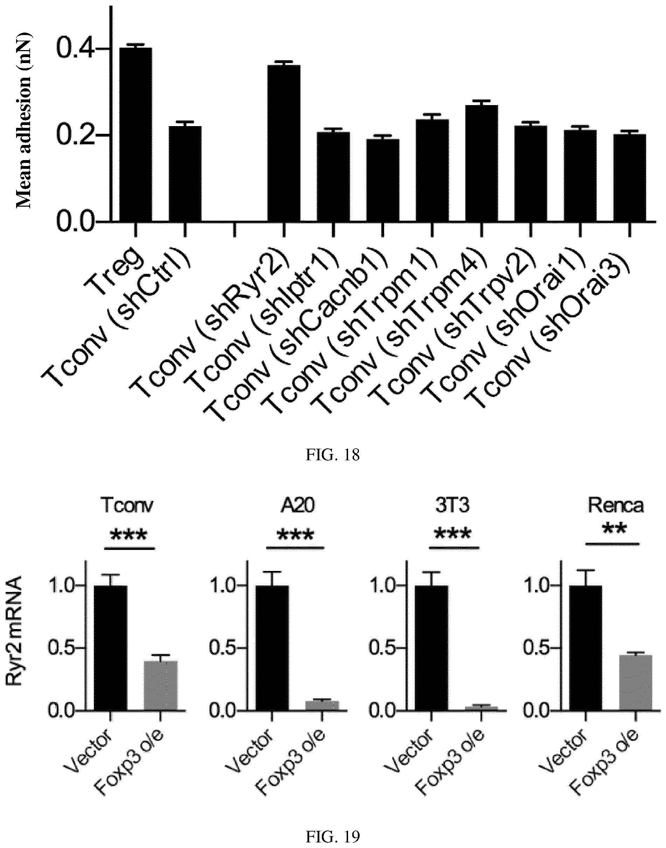

FoxP3 was first overexpressed in T cells, B cells (A20), 3T3 and Renca tumor cells (using the method in Example 1). In these four cases, FoxP3 overexpression significantly blocked the transcription of Ryr2 gene, indicating that FoxP3 spontaneously targets Ryr2 (FIG. 19).

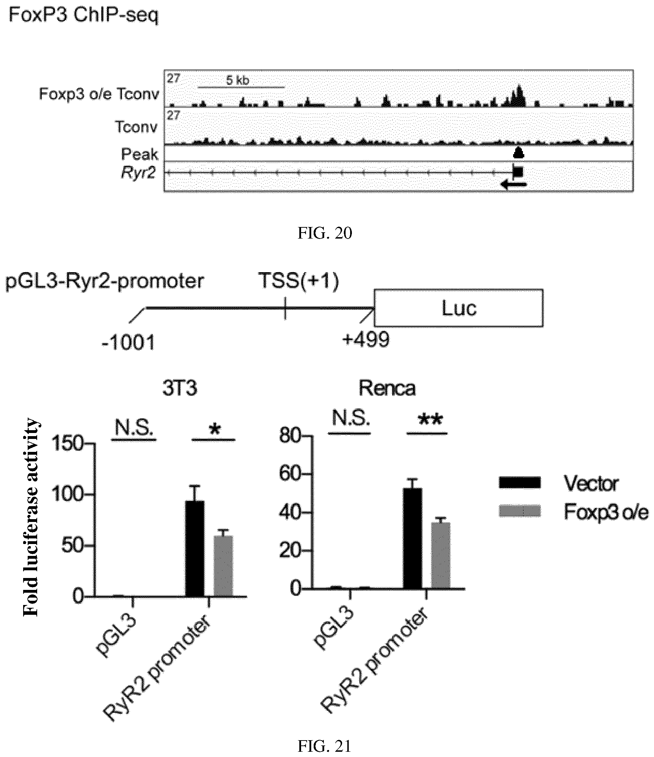

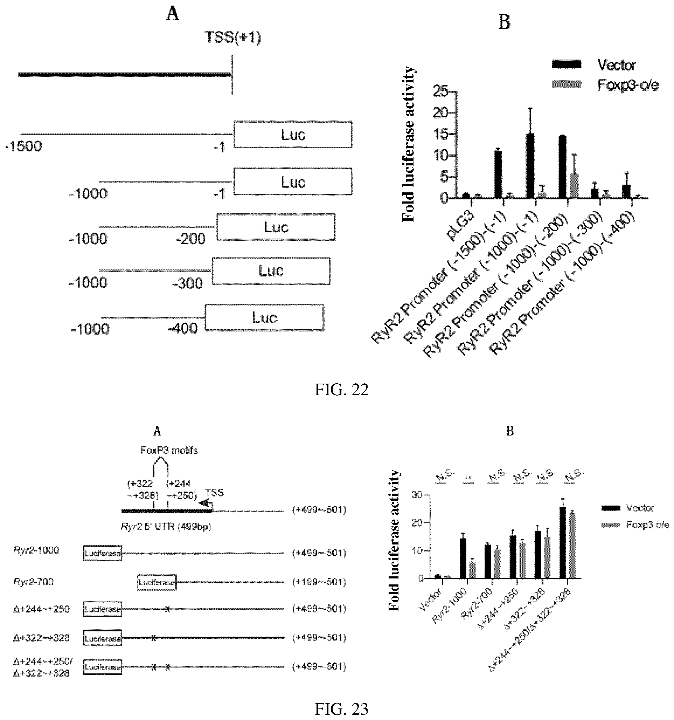

Then, a genome-wide Chip-seq data set for gene expression analysis was prepared, wherein the ChIP-seq data was downloaded from the GEO data set, and GEO ID was as follows: Foxp3 in Tconv GSM989036, and FoxP3 in Tconv cells was transduced to expression marker-FoxP3 GSM989034. IGV (v2.4.14) facilitated visualization of ChIP-seq data using the mouse reference genome mm8. As shown in the screenshots at specific gene sites, when the data set was concentrated within a 40-kb region around the Ryr2 gene using the IGV program, a strong signal was detected within the 1.5-kb promoter region in the untransfected control group (FIG. 20). Furthermore, the above regions were cloned, and expression vectors of luciferase reporter genes were constructed under the control of this promoter for expression in 3T3 and Renca cells, including the FoxP3 overexpression group and the non-FoxP3 overexpression group. As can be seen in FIG. 21, FoxP3 overexpression reduced luciferase activity. Then, sequential segment deletion was performed in the sequence (see FIG. 22A). In a series of experiments, the inhibitory activity was gradually narrowed to a restricted region of about 300 bp to 500 bp after the TSS site, and 200 bp before the start codon of Ryr2 (FIG. 22B).

The FoxP3 binding site was finally determined as the two GCAGGGG sequence repeats. When these two sites were mutated, FoxP3 overexpression lost the ability to inhibit luciferase, indicating that it is the binding site for FoxP3 in this promoter (FIG. 23). Meanwhile, in order to further confirm that inhibition of luciferase activity is associated with Ryr2 activity, this example triggered Ryr2-mediated Ca²⁺ throughput in 3T3 and A20 cells, including the FoxP3 overexpression group and the non-FoxP3 overexpression group. FIG. 24 shows that 4-CmC drives much less Ca²⁺ signal in FoxP3-transfected cells under the action of FoxP3, indicating that Ryr2 is indeed under direct control of FoxP3, and it is a key in T cell inhibitory activity. Among them, the fold luciferase reporter assay procedures involved in this example is described in the documents "Identification and characterization of MAVS, a mitochondrial antiviral signaling protein that activates NF-kappaB and IRF 3" (Seth et al., Cell 122, 669-682, 2005) and "LFA-1-mediated adhesion is regulated by cytoskeletal restraint and by a Ca2+-dependent protease, calpain" (Stewart et al., J Cell Biol, 140, 699-707, 1998). The mouse Ryr2 reporter plasmids were constructed by subcloning the Ryr2 promoter of 1500 bp into the luciferase expressing pGL3 vectors. The complete promoter sequence and the truncated promoter were synthesized, and the plasmids were confirmed correct by sequencing. 1.25 × 10⁵ FoxP3-overexpressed 3T3, FoxP3-overexpressed Renca or FoxP3-overexpressed A20 cells were co-transfected with 300 ng of Ryr2 reporter gene and ranilla luciferase reporter plasmids using Neofect (KS2000). 36 h after transfection, cell lysates were prepared and analyzed using the fold luciferase reporter assay system (Promega).

### Example 3: Effect of Ryr2 inhibition on Ca²⁺ levels in T cells

### 1. M-calpain differences in Tconv and Treg, and binding ability to DC

Since the m-calpain activity is low in Treg, this example is expected to determine whether the expression level is low or not. Meanwhile, m-calpain is regulated by intracellular Ca²⁺ availability. Therefore, Ca²⁺ signals of Tconv and Treg in the resting state were detected.

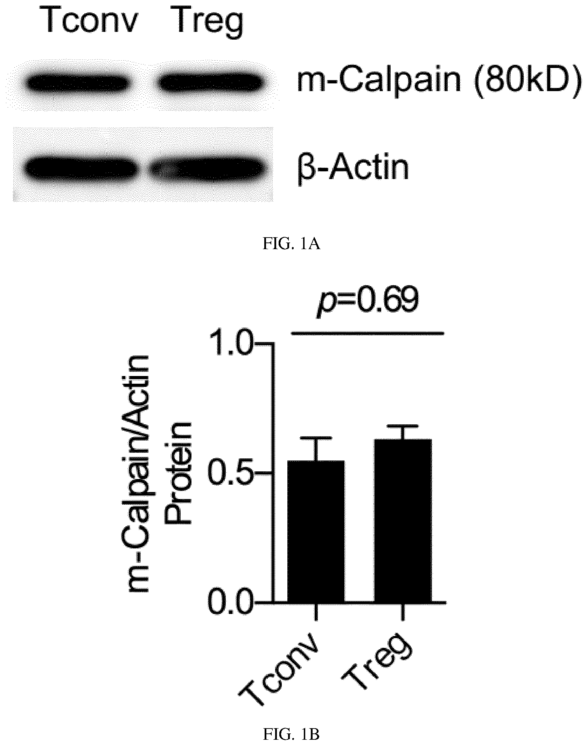

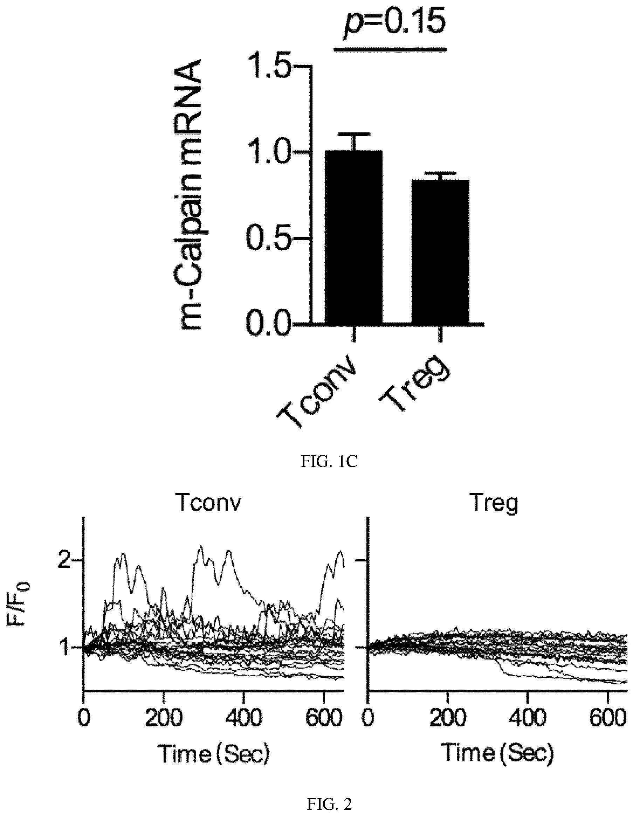

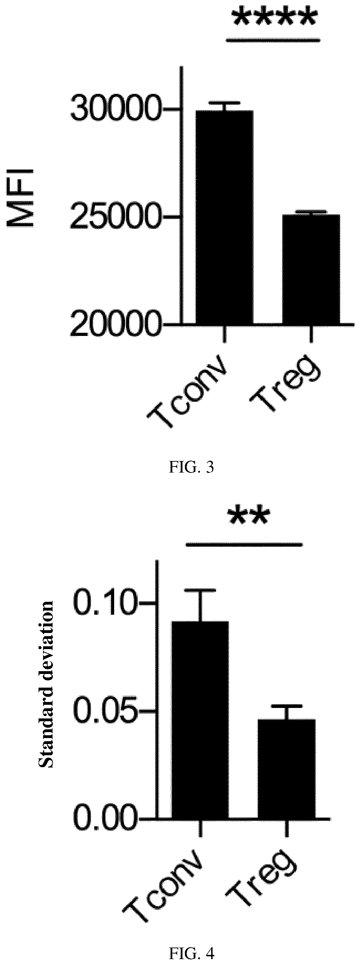

The results are shown in FIGs. 1A, B and C, wherein m-calpain in Tconv and Treg demonstrated no difference at protein or mRNA level. Although basal Ca²⁺ fluctuations are a common feature shared by many immune cells including Tconv, this activity was essentially absent in Treg. That is, the peak of Ca²⁺ oscillations were not found in individual Treg (FIGs. 2 and 3). The standard deviation of Ca²⁺ fluctuation intensity in Treg was significantly less than that in Tconv (FIG. 4). This indicated that m-calpain activity was blocked in Treg, providing it an unusual strong binding ability to DC based on LFA-1/icam-1.

### 2. Determination of factors affecting Ca²⁺ levels

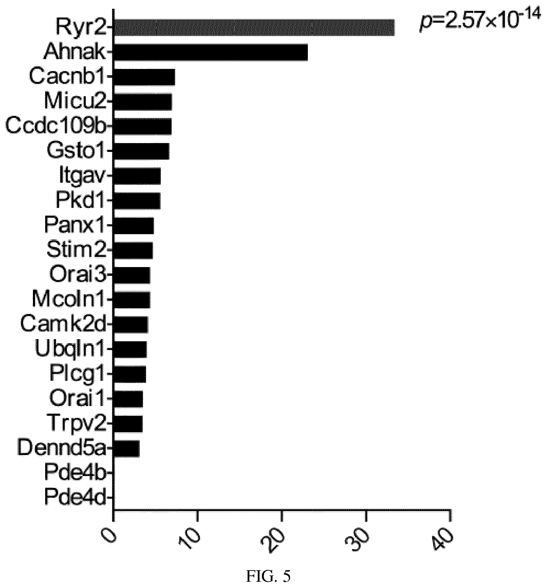

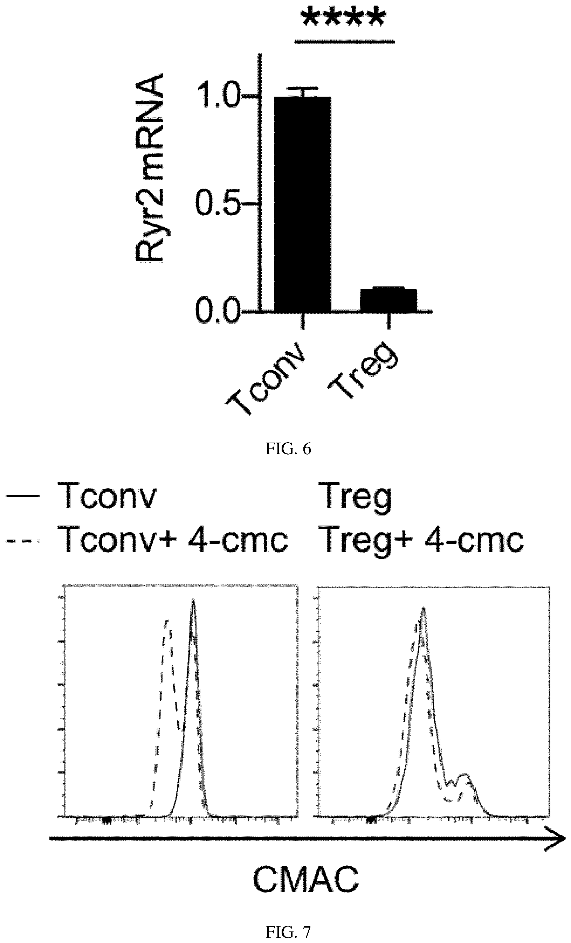

Upon T cell activation, Ca²⁺ signals were from the IP3R channel as the result of an expansion of the immunotyrosine-based signaling cascade to a secondary Ca²⁺ amplification event. However, this activation-induced signal was absent in resting cells. In this example, the data of several factors affecting Ca²⁺ signal regulation involved in the database GEO DataSets (see "A mechanism for expansion of regulatory T-cell repertoire and its role in self-tolerance" (Feng et al., Nature, 2015)) were used, and the details are shown in FIG. 5. The reason for the low basal Ca²⁺ level in Treg was verified and sorted according to the difference degree of the Treg expression and Tconv expression. The results are shown in FIG. 5, wherein Ryr2 had the largest difference, and the expression level in Tconv was 5-fold log2 or more (32-fold linear) higher than that in Treg, with a p value of 2.57 × 10⁻¹⁴. Further analysis using qPCR confirmed this conclusion (see FIG. 6).

The GEO database analysis comprised the following steps: proteins were classified as per expression level according to the three RNA-Seq studies published in NCBI GEO database (GSE71162). Calcium-associated proteins were sorted by GO analysis. The functions of these proteins were annotated based on UniProt (http://www.uniprot.org/). The proteins were sorted by the degree of expression difference between Treg and Tconv.

Ryr receptors are located on the er membrane (Ryr1, 2 and 3). In human PBMC, Ryr1 is expressed in the CD19+ population and Ryr2 is expressed in the CD3+ fraction. In order to confirm that Ryr2 is expressed in Treg in low amounts, the digestion rate of CMAC (calpain substrate) was observed using the stimulatory factor 4-CMC. The results are shown in FIG. 7, 4-CMC is completely able to induce calpain activity, whereas Treg is not sensitive to this treatment, confirming the reduced expression of Ryr2 in Treg.

### 3. Inhibiting Ryr2 in Tconv cells can reduce Ca²⁺ levels and basal oscillations

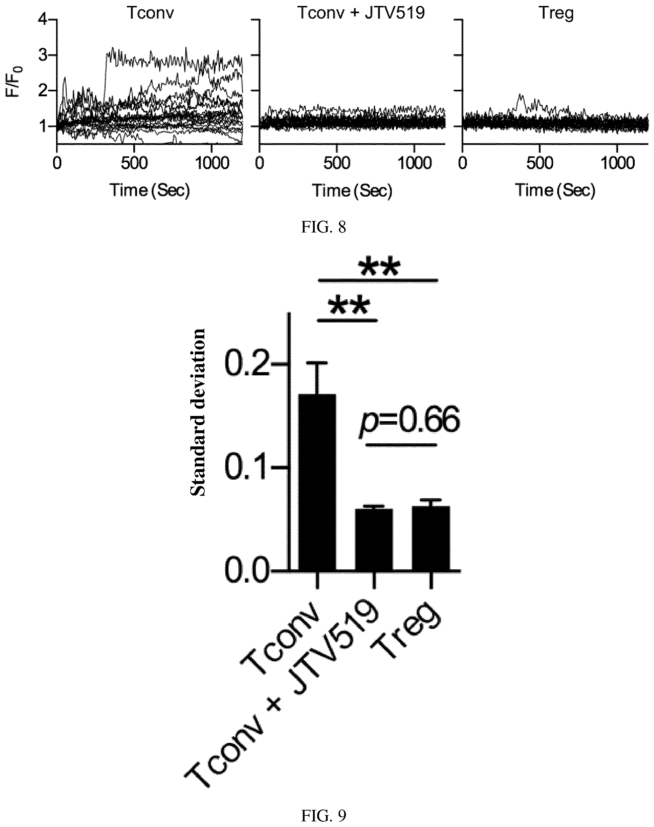

This example further verified the change in Ca²⁺ signal after Ryr2 inhibition, and the results are as shown in FIG. 8 and 9. The Tconv cells after Ryr2 inhibition had similar Treg effects regardless of the peak value or the standard deviation of Ca²⁺ signal, and the calcium ion level was significantly reduced compared to Tconv cells without Ryr2 inhibition.

In summary, the lower Ca²⁺ levels in the T cell subpopulation were essentially controlled by the inhibition of Ryr2 transcription.

### Example 4: Blocking Ryr2 can tightly bind T Cells to DCs

### 1. Ryr2 knock down in Tconv can reduce Ca²⁺ levels

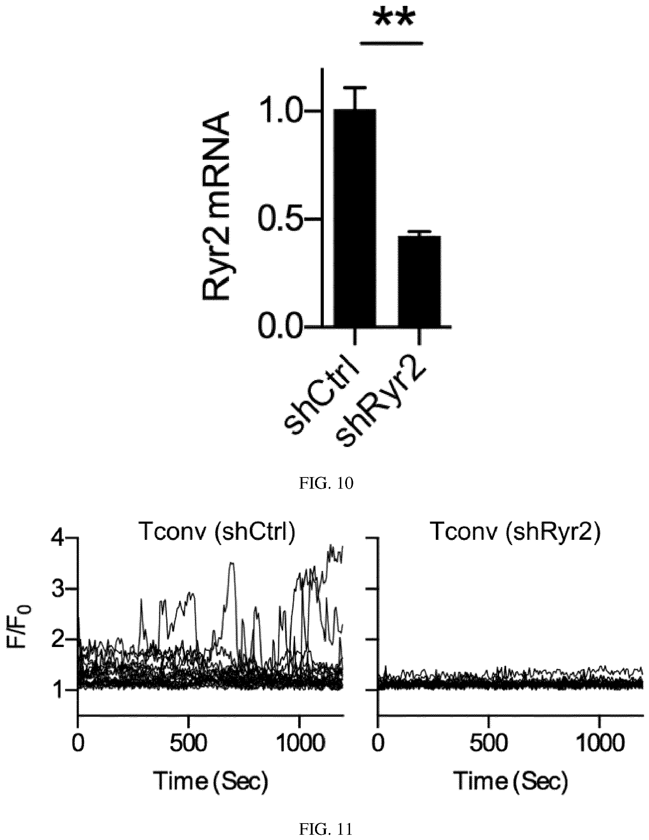

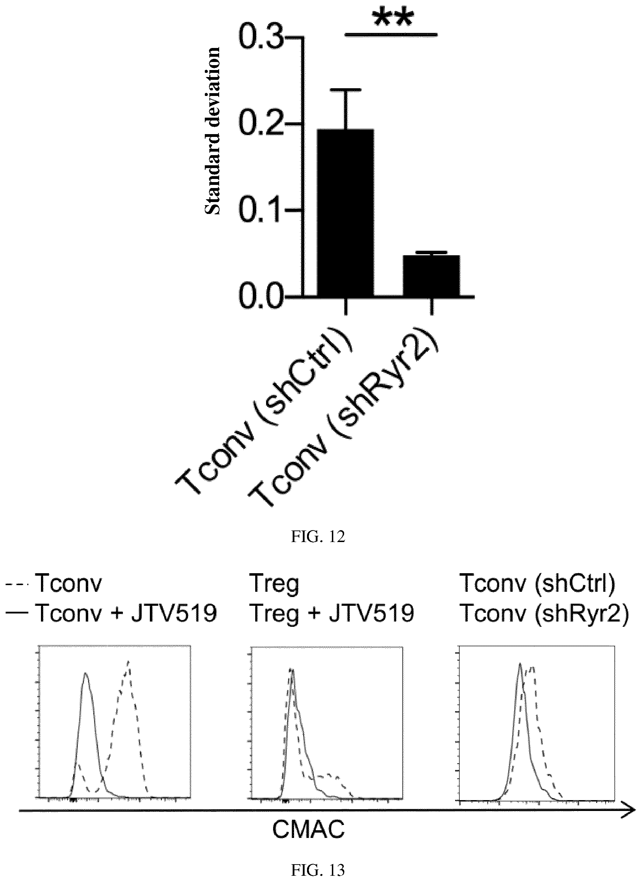

In order to genetically confirm that Ryr2 inhibition is responsible for the reduction of Ca²⁺ levels in Tconv, shRNA interference for knock down of Ryr2 was performed in this example. Transcriptional mRNA levels were compared between group with Ryr2-specific knock down by shRNA and group without Ryr2 knock down (FIG. 10). Tconv with Ryr2 knock down had significantly reduced cellular basal Ca²⁺ levels, as compared to control group (FIGs. 11 and 12). After knock down, the Ca²⁺ peak was reduced, and the standard deviation of Ca²⁺ change was also reduced, which was consistent with the results of the digestion of low m-calpain substrate, e.g., inhibition Ryr2 after JTV519 treatment (FIG. 13).

Wherein calpain activity was measured. To measure calpain activity, 10⁵ T cells were incubated in 200 µL of medium containing 20 µM calpain substrate CMAC (t-BOC-Leu-Met, Thermo Fisher) in the dark at room temperature. After 5 min of incubation, the reaction was terminated with 1% PFA (Biosharp) and the cells were immediately placed on ice. The fluorescent signal emitted from the digested substrate was measured as calpain activity by Fortech flow cytometer through the Hoechst blue channel. In all experiments, the inhibitor JTV519 was added 30 min prior to the experiments, and activator 4-CmC and CMAC were added at the same time.

### 2. Effect of Ryr2 deletion in Tconv cells on T cells binding to DCs

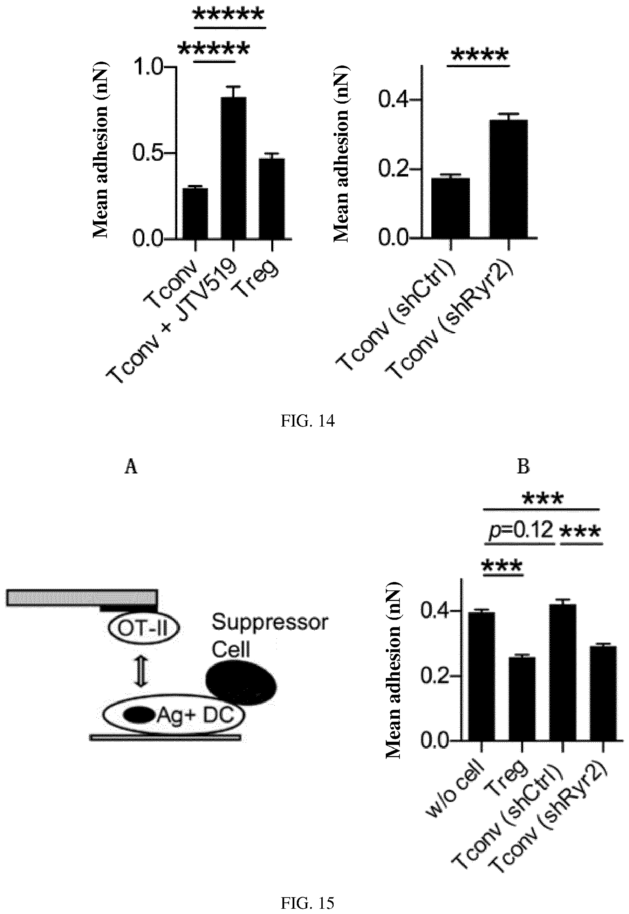

To verify the effect of the reduction in Ryr2 and loss of m-calpain activity on T cells binding to DCs, in this example, shRNA and JTV519 were used to block Ryr2 in Tconv. The results showed that both treatments induced strong contact with dendritic cells, and the intensities were even stronger than that of Treg (FIG. 14).

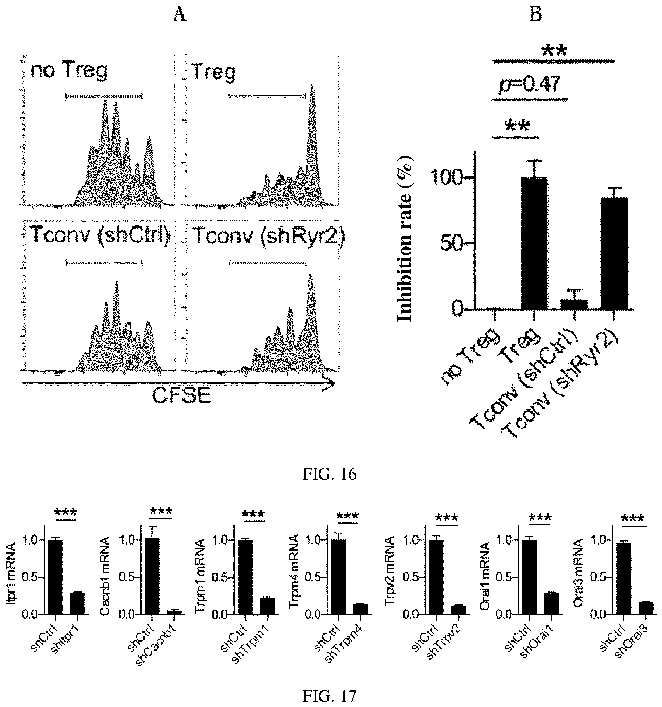

Furthermore, it was confirmed that improved binding of treated Tconv to DC resulted in stable binding of antigen-specific Tconv to the same DC, and a triple cell binding force analysis was conducted (see: "Strong adhesion by regulatory T cells induces dendritic cell cytoskeletal polarization and contact-dependent lethargy" (Chen et al., The Journal of experimental medicine, 2017)). The results showed that Treg-mediated blocking, and Ryr2-reduced Tconv can significantly inhibit the binding strength between OTII and OVA-loaded DC (FIG. 15). Therefore, it was confirmed at the single cell level that Tconv with reduced Ryr2 activity was comparable to Treg function in its ability to prevent Tconv interaction with DC. shRNA-treated Tconv was then tested for its ability to inhibit OTII activation in a typical DC-mediated antigen presentation assay. The results are shown in FIG. 16, These cells exhibited a similar function to Tregs in reducing OTII division. In an investigation on other potential Ca²⁺ channel activities that may function in a similar manner to Ryr2, a set of shRNA was generated to reduce their activities in CD4 Tconv cells, and their binding to DCs was tested by SCFS. The results are shown in FIGs. 17 and 18. None of the knock downs other than Ryr2 improved the binding of treated T cells to DCs, confirming the unique involvement of Ryr2.

The functions were inhibited *in vitro.* Purified OTII T cells were stained with CellTrace CFSE (Thermo Fisher Scientifc). 10⁴ purified DCs from spleen cells, 2 × 10⁴ OTII T cells, 2 × 10- Treg or Ryr2-/- Tconv or Ryr2-knock down Tconv and 2 µM OVA323-339 peptides were mixed and cultured in each well of a 96-well U-bottom plate, and the mixture was evaluated for proliferation of OTII T cells by CFSE dilution using Fortessa flow cytometer. Inhibition percentage was calculated by (1 - proliferation%), and then the data was normalized by taking the Treg-free group as 0% inhibition and the Treg group as 100% inhibition.

### 3. The confirmation of binding to DCs in the deletion of Ryr2 using force spectrum

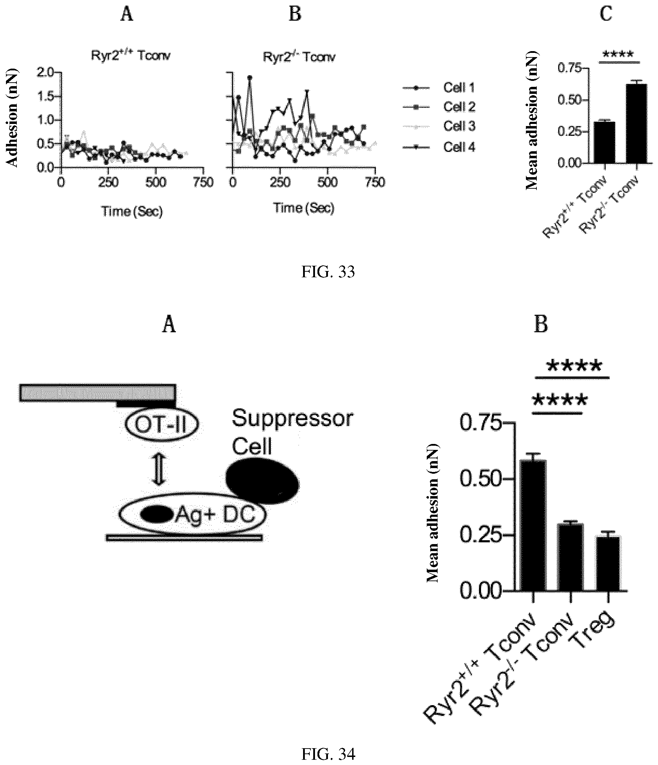

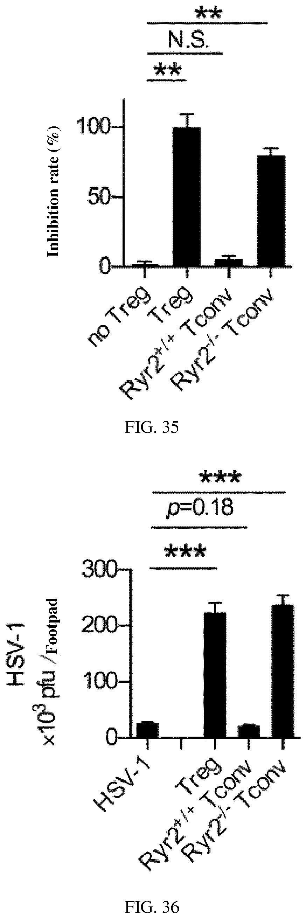

Similarly, the force spectrum also showed improved binding to DCs when Ryr2 was deleted (FIG. 33). At the same time, the ability to interfere with DCs contact with T cells was also enhanced in the triple-cell force assay (FIG. 34). More importantly, Ryr2-/- Tconv was similar to Treg in its ability to inhibit the amplification of antigen-positive dendritic cell-stimulated OTII (FIG. 35). Therefore, the ability to inhibit DC-mediated T cell activation could be obtained *in vitro* by simply limiting Ryr2 in Tconv cells. FIG. 46 shows the immunosuppressive effect of T cells with inhibition of Ryr2.

The atomic force microscope-based single cell force spectrum procedures were performed using JPK CellHesion device as described above (see "Alum interaction with dendritic cell membrane lipids is essential for its adjuvanticity" (Flach et al., Nat Med 17, 479-487, 2011) and "Strong adhesion by regulatory T cells induces dendritic cell cytoskeletal polarization and contact-dependent lethargy" (Chen et al., The Journal of experimental medicine, 2017)). Briefly, to measure T-DC adhesion in a two-cell system, DC2.4 cells were cultured on untreated glass plates. T cells were treated with 200 U/mL human IL-2 overnight. The disks were moved into an AFM compatible chamber and mounted on a machine table. Clean cantilevers were coated with CellTak (BD), and then attached to an individual T cell on the disk. The AFM cantilever carrying the individual T cell was lowered to allow T cell contact with a single DC and to interact for 15 s, and then moved upward until the two cells were completely separated. A force curve was obtained. The process was then repeated. For the triple cell system, DC2.4 cells cultured on glass plates were treated with 100 µg/mL soluble OVA protein for 4 h prior to the experiment. IL-2 treated Treg/Tconv cells were stained with 10 µM CFSE, then DC2.4 cells were incubated with these fluorescently labeled Treg or Tconv cells for about 20 min, followed by addition of unlabeled OT-II T cells. The Treg/Tconv cell-DC pair identified with the UV flash lamp was then approached by a cantilever tip carrying OT-II T cells. Treg/T transformation-mediated inhibition of OT-II-DC adhesion was analyzed. In each cycle, the AFM cantilever carrying an individual T cell was lowered in 0.5 µm to 2 µm increments until the first force curve was generated. The T cells on the cantilever were then allowed to interact with the DCs for 15 s, and then moved upward until the two cells were completely separated. The incubator containing the device was set at 37 °C and 5% CO₂. In all experiments, at least 14 force curves were collected for further analysis. The force curves were processed using JPK image processing software. Only round and robust cells were selected for AFM gluing. For each SCFS experiment, a T-DC pair was used to generate force readings for each up-down cycle during a period of several minutes. At least three pairs of data were used for each condition.

### Example 5: Effect of Ryr2-/- T cells in HSV-1 infection

### 1. Preparation of herpes infection model and testing procedures

Herpes infection was induced by 10⁶pfu HSV-1 (strain F) in 20 µL PBS into hinder footpads. Cells were adoptively transferred into the footpadat 3 dpi with 2 × 10⁵ cells/20 µL PBS, Treg, or Ryr2-/- Tconv or Ryr2+/+ Tconv. The homogenized footpad tissue was tested for viral titer at 7 dpi. Each group contained 4-6 mice and the experiment was repeated 3 times.

For DTH, the right footpad was re-challenged with UV-inactivated HSV-1 (10⁶pfu/20 µL PBS) at 6 dpi, and the footpad swelling was then measured at 7 dpi with the left footpad as a control. Each group contained 16-21 mice.

### 2. Results

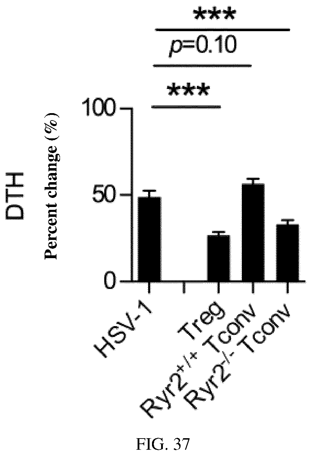

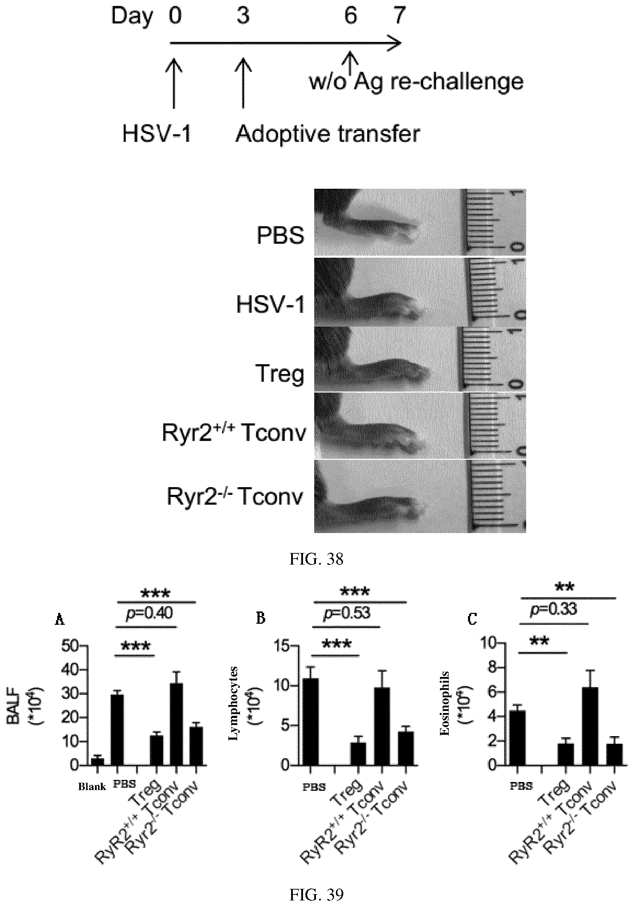

In the herpes infection model, HSV-1 pfu counts began to increase at the approximately 1 log injection of Treg or Ryr2-/- Tconv. The infusion of Ryr2+/+ Tconv was not increased as compared to viral inoculation alone (PBS) (see FIG. 36). The same model was then used to detect DTH responses. After 2 inoculations with HSV-1, allergic swelling was found in footpad, but the increase in the thickness was reduced with injection of Treg and Ryr2-/- Tconv, whereas the increase in the thickness was not decreased in control group Ryr2+/+ Tconv and group with viral inoculation alone (PBS) (FIGs. 37 and 38).

### Example 6: Effect of Ryr2-/- T cells in a mouse model with asthma

### 1. Preparation of asthma model and testing procedures

Ovalbumin (OVA) can induce airway inflammation. Mice were sensitized on day 0 and day 14 by two *i.p.* injections of OVA with alum adjuvant (100 µg + 4 mg). Intra-tracheal OVA re-challenge were repeatedlygiven at back of the tongue on day 21, day 23 and day 25. 10⁶ Treg, Ryr2-/- Tconv or Ryr2+/+ Tconv, or PBS were adoptively transferred by i.v. BALF infiltration and histology were analyzed on day 32.

### 2. H&E histological procedures

Skin, ear, liver and other tissues were all immobilized in 4% nerve-buffered formalin at 4 °C. The samples were then embedded in paraffin. Glass slides were cut into slides of five to six microns in thickness. All glass slides were stained with hematoxylin and eosin.

### 3. Results

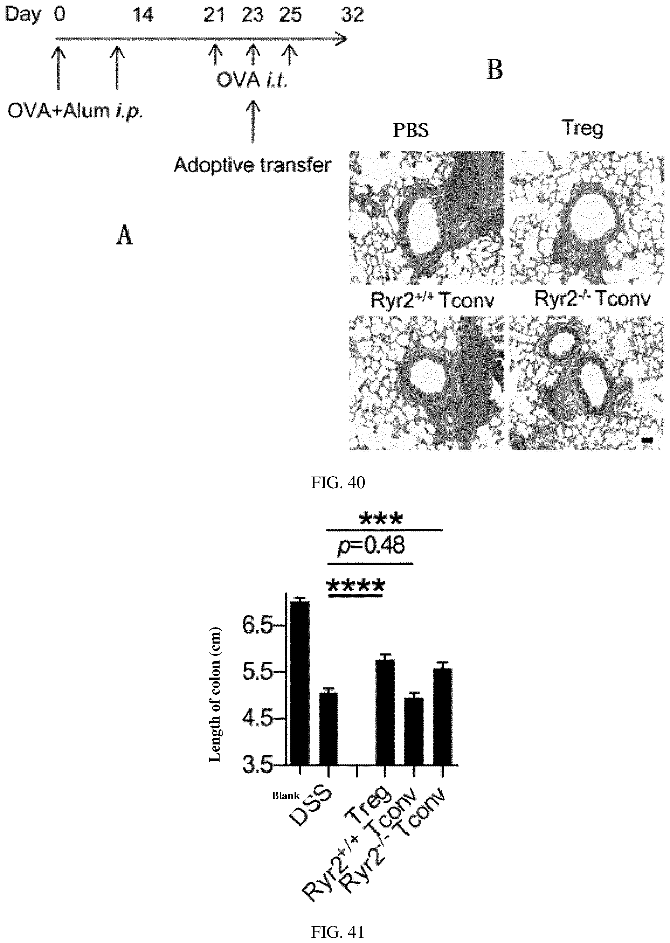

Infusion of Treg and Ryr2-/- Tconv was equally effective in limiting the number of BALF cells in an OVA-sensitized asthma model generated according to the sensitization schedule shown in FIG. 40A, with similar reduction in lymphocytes and eosinophils (FIG. 39). Histological detection is shown in FIG. 40B.

### Example 7: Effect of Ryr2-/- T cells in a mouse model with inflammatory bowel disease

### 1. Preparation of DSS-induced inflammatory bowel disease (IBD) model and testing procedures

The DSS-induced mouse experimental inflammatory bowel disease model can be seen in the documents: "Inhibition of Dectin-1 Signaling Ameliorates Colitis by Inducing Lactobacillus-Mediated Regulatory T Cell Expansion in the Intestine" (Tang et al., Cell Host Microbe 18, 183-197, 2015) and "Myeloid-Derived Suppressor Cells Are Controlled by Regulatory T Cells via TGF-beta during Murine Colitis" (Lee et al., Cell 17, 3219-3232, 2016). Briefly, wild-type Tconv or Treg were transferred into 6-week-old male C57BL/6 mice by i.v. adoptive transfer of 3 × 10⁶ Ryr2-/- Tconv. The next day, colitis was induced by oral administration of 4% DSS (w/v) (Yeason, MW = 36,000 Da to 50,000 Da) for 7 days, and then the mice was fed with ordinary drinking water. Normal control mice were treated with PBS and given normal drinking water. Mice were sacrificed on day 10, the colon was dissected and the colon length was measured. The colon was then immobilized in 4% PFA at 4 °C within 48 h for subsequent H&E staining. The colon sections were observed by 3DHISTECH Pannoramic SCAN (3DHISTECH). Each group contained 11-18 mice.

### 2. H&E histological procedures

Skin, ear, liver and other tissues were all immobilized in 4% nerve-buffered formalin at 4 °C. The samples were then embedded in paraffin. Glass slides were cut into slides of five to six microns in thickness. All glass slides were stained with hematoxylin and eosin.

### 3. Results

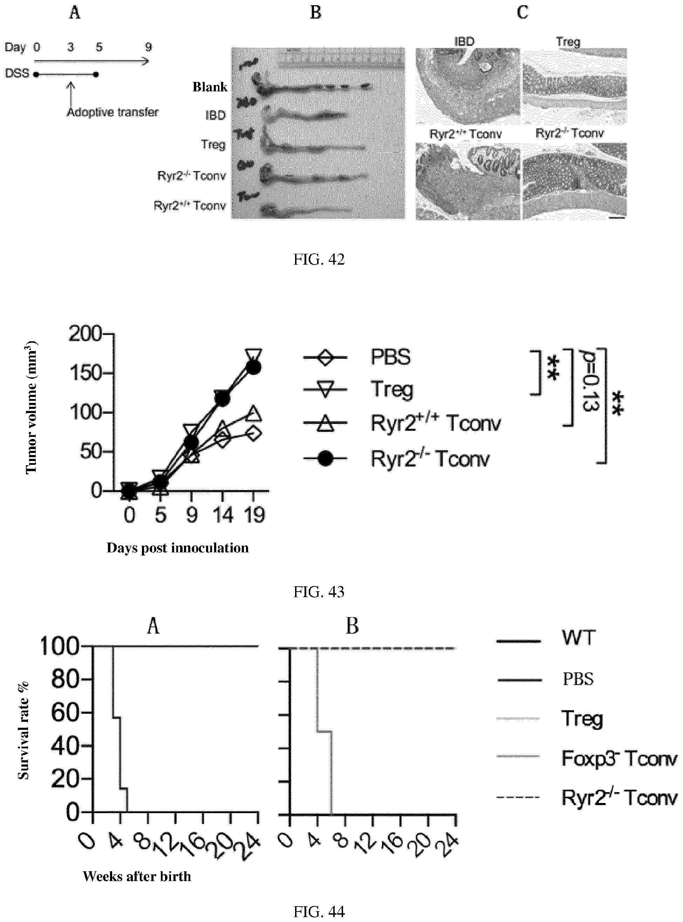

In the colitis model generated according to the induction schedule shown in FIG. 42A, the Treg and Ryr2-/- Tconv-injected groups demonstrated reduced length of colon. Both Treg and Ryr2-/- Tconv groups had reduced colonic damage as compared to Ryr2+/+ Tconv group (FIG. 41 and 42).

### Example 8: Effect of Ryr2-/- T cells in a mouse colon cancer model

### 1. Preparation of colon cancer model and testing procedures

The mouse model with the transplanted tumor could be established by reference to the "Oxidative stress controls regulatory T cell apoptosis and suppressor activity and PD-L1-blockade resistance in tumor" (Maj et al., Nat Immunol 18, 1332-1341, 2017). Briefly, MC38 colon cancer cells were washed twice in PBS and then 5 × 10⁵ MC38 cells and 10⁶ T cells in 200 µL PBS were injected subcutaneously into the abdomen of 6-week-old male C57BL/6 mice. Tumor growth was monitored using a vernier caliper every 4-5 days from day 7 after tumor injection. The volume was calculated as (length × width × width) / 2. Group 1: MC38 + PBS; group 2: MC38 + Ryr2+/+ Tconv; group 3: MC38 + Treg; group 4: MC38 + Ryr2-/- Tconv. Each group contained ten mice.

### 2. Results

In the MC38 tumor model, Treg and Ryr2-/- Tconv injection promoted the tumor growth, while no significant effect was seen in PBS and Ryr2+/+ Tconv groups (FIG. 43).

### Example 9: Immune homeostasis restoring effect of Ryr2-/- T cells in a mouse psoriasis model

Ryr2-/- Tconv cells play the most important role in general inflammation caused by multi-organ autoimmunity. Therefore, an Foxp3-deficient mouse model was constructed in this example to verify immune homeostasis restoring under the regulation of Ryr2-/- Tconv cells in FoxP3-deficient immunodeficiency mice.

### 1. Psoriasis mouse (Scurfy) model

The Foxp3-deficient psoriasis mouse model was established with reference to the "A requisite role for induced regulatory T cells in tolerance based on expanding antigen receptor diversity" (Haribhai et al., Immunity 35, 109-122, 2011) and "TGF-beta-induced Foxp3+ regulatory T cells rescue scurfy mice" (Huter et al., Eur J Immunol 38, 1814-1821, 2008). 5 × 10⁶ T cells were purified, washed twice in PBS and resuspended in 50 µL PBS for intraperitoneal injection. The injection was performed on day 2 or day 3 after birth of newborn homologous psoriasis mice. Adoptive transfer of T cells was performed every three or four days in the first two weeks, and then performed every two weeks. At adoptive transfer, body weights of the psoriasis model mice and male WT littermates were recorded and the survival rates were monitored. All mice were sampled on the day of birth and genotyped for the sf mutant gene by PCR, and their genotypes were confirmed by sequencing. FoxP3 PCR primers were 5'-CATCCCACTGTGACGAGATG-3' (SEQ ID NO: 1) and 5'-ACTTGGAGCACAGGGGTCT-3' (SEQ ID NO: 2). For histology, mice receiving PBS were analyzed at week 3, and mice adoptively transferred Treg and Ryr2-/- Tconv were analyzed at weeks 8-12.

### 2. H&E histological procedures

Skin, ear, liver and other tissues were all immobilized in 4% nerve-buffered formalin at 4 °C. The samples were then embedded in paraffin. Glass slides were cut into slides of five to six microns in thickness. All glass slides were stained with hematoxylin and eosin.

### 3. Results

2-3 days after birth, psoriasis mice (FoxP3-/-, C57BL/6) were injected with PBS, Treg, Ryr2+/+ Tconv or Ryr2-/- Tconv, respectively. All mice receiving PBS or Ryr2+/+ Tconv died within 2-4 weeks after birth as expected. In contrast, all mice receiving Treg or Ryr2-/- Tconv survived for at least 20 weeks (FIGs. 44 and 25). Moreover, after observing for 6 months, none of Ryr2-/- transfused psoriasis mice has died or shown any overt abnormality.

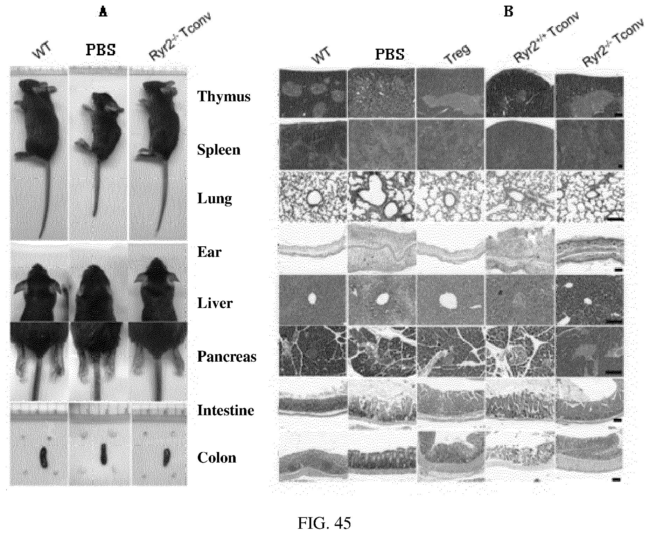

The histological results are shown in FIG. 45. As expected, the mice receiving PBS or Ryr2+/+ Tconv exhibited severe thyroiditis, splenitis, pneumonia, dermatitis, hepatitis, pancreatitis, gastritis and colitis. However, the mice receiving Treg or Ryr2-/- Tconv were completely treated for these pathologies. That is, the loss of comprehensive effector function was restored in FoxP3-deficient mice, indicating that Ryr2 modulates T cell immune function.

In conclusion, Ryr2-/- T and Treg cells all showed functional equivalence in the mouse model with herpes infection, the asthma model, the colitis model and the colon cancer model, and can also restore the immune function of immunodeficiency mice. That is, Ryr2-/- T may all play an equivalent role in some diseases where Treg are known to have immunoregulation effects.

The preferred embodiments of the present invention are described in detail above, which, however, are not intended to limit the present invention. Within the scope of the technical concept of the present invention, various simple modifications can be made to the technical solution of the present invention, all of which will fall within the protection scope of the present invention.In addition, it should be noted that the various specific technical features described in the above specific embodiments can be combined in any suitable manner without contradiction. In order to avoid unnecessary repetition, such combinations will not be illustrated separately.

## Claims

1. A T cell, wherein Ryr2 gene is deleted in the T cell.

2. The T cell according to claim 1, wherein exon 7 of the Ryr2 gene is deleted in the T cell.

3. The T cell according to claim 1, wherein at least a guanine-rich sequence in the Ryr2 gene is deleted in the T cell; preferably, the guanine-rich sequence is located within first 200 bp from a start codon of the Ryr2 gene.

4. The T cell according to claim 3, wherein the guanine-rich sequence comprises GCAGGGG.

5. The T cell according to any one of claims 1 to 4, wherein the T cell is a Tconv cell.

6. A method for preparing the T cell according to any one of claims 1 to 5, wherein the method is selected from shRNA, siRNA, CRISPR/Cas9, zinc finger nuclease technology, transcription activator-like effector nuclease technology or meganuclease.

7. The method according to claim 6, wherein the shRNA is set forth in SEQ ID NOs: 5 or 6.

8. An shRNA, wherein the shRNA knocks down Ryr2 gene of a T cell, thus reducing basal Ca²⁺ oscillation in the T cell, reducing m-calpain activity in the T cell, improving binding strength T cells to DC cells, allowing the T cell to have functions of immunosuppressive cells or functions of treating an infectious disease, an inflammation or a tumor.

9. The shRNA according to claim 8, wherein the shRNA is set forth in SEQ ID NOs: 5or 6.

10. A T cell, wherein Ryr2 is overexpressed in the T cell.

11. The T cell according to claim 10, wherein the T cell is a Treg cell.

12. Use of a reagent for regulating Ryr2 expression, a reagent for regulating basal Ca²⁺ oscillation, a reagent for regulating m-calpain activity, a reagent for regulating binding strength of a T cell to a DC cell, or the T cell according to any one of claims 1 to 5, 10 and 11 in preparing a medicament for treating an infectious disease, an inflammation or a tumor.

13. Use of a reagent for regulating Ryr2 expression, a reagent for regulating basal Ca²⁺ oscillation, a reagent for regulating m-calpain activity, a reagent for regulating binding strength of a T cell to a DC cell, or the T cell according to any one of claims 1 to 5, 10 and 11 in treating an infectious disease, an inflammation or a tumor.

14. Use of a reagent for reducing Ryr2 expression, a reagent for reducing basal Ca²⁺ oscillation, a reagent for reducing m-calpain activity, a reagent for improving binding strength of a T cell to a DC cell, or the T cell according to any one of claims 1 to 5 in preparing a medicament for treating an infectious disease or an inflammation.

15. Use of a reagent for reducing Ryr2 expression, a reagent for reducing basal Ca²⁺ oscillation, a reagent for reducing m-calpain activity, a reagent for improving binding strength of a T cell to a DC cell, or the T cell according to any one of claims 1 to 5 in treating an infectious disease or an inflammation.

16. Use of a reagent for improving Ryr2 expression, a reagent for improving basal Ca²⁺ oscillation, a reagent for improving m-calpain activity, a reagent for reducing binding strength of a T cell to a DC cell, or the T cell according to claim 10 or 11 in preparing a medicament for treating a tumor.

17. Use of a reagent for improving Ryr2 expression, a reagent for improving basal Ca²⁺ oscillation, a reagent for improving m-calpain activity, a reagent for reducing binding strength of a T cell to a DC cell, or the T cell according to claim 10 or 11 in treating a tumor.

18. The use according to any one of claims 12 to 15, wherein the infectious disease is selected from a bacterial infection, a viral infection or a fungal infection, preferably selected from a viral infection, pneumonia with septic shock, peritonitis, bacteremia, sepsis or septicemia; the viral infection is selected from an acute viral infection or a chronic viral infection, and the virus is preferably selected from influenza virus, parainfluenza virus, herpes virus, measles virus, vesicular stomatitis virus, hepatitis B virus, hepatitis C virus, human immunodeficiency virus, lymphocytic choriomeningitis virus or human papilloma virus.

19. The use according to any one of claims 12 to 15, wherein the inflammation is selected from systemic lupus erythematosus, rheumatoid arthritis, psoriatic arthritis, scleroderma, asthma, atopic dermatitis, organ-specific inflammatory diseases, allergies, folliculitis, tonsillitis, pneumonia, hepatitis, nephritis, acne, autoimmune diseases, chronic prostatitis, glomerulonephritis, hypersensitivity reactions, colitis, inflammatory bowel diseases, pelvic inflammatory disease, reperfusion injury, transplantation rejection, vasculitis or interstitial cystitis.

20. The use according to any one of claims 12, 13, 16 and 17, wherein the tumor is selected from prostate cancer, breast cancer, liver cancer, glioma, intestinal cancer, cervical cancer, non-small cell lung cancer, lung cancer, pancreatic cancer, gastric cancer, bladder cancer, skin cancer, striated muscle cancer, tongue squamous carcinoma, nasopharyngeal cancer, ovarian cancer, placental choriocarcinoma, lymphoma, leukemia, rectal adenocarcinoma, medulloblastoma, meningioma, neurofibroma, ependymoma, schwannoma, astrocytoma, melanoma, mesothelioma, myeloma, chronic granulocytic leukaemia, acute myelogenous leukemia, myelodysplastic syndrome, chronic lymphocytic leukemia, epidermoid cancer, colon cancer, thymus cancer, blood cancer, head or neck cancer or oropharyngeal cancer.

21. Use of regulation of Ryr2 expression in regulating basal Ca²⁺ oscillation, regulating m-calpain activity or improving binding strength of a T cell to a DC cell.

22. Use of regulation of basal Ca²⁺ oscillation in regulating m-calpain activity or regulating binding strength of a T cell to a DC cell.

23. Use of regulation of m-calpain activity in regulating binding strength of a T cell to a DC cell.

24. The use according to any one of claims 21 to 23, wherein the regulation is a reduction or an improvement.

25. An Ryr2 antagonist, wherein the antagonist targets exon 7 of the Ryr2 gene or a guanine-rich sequence in the Ryr2 gene; preferably, the guanine-rich sequence is located within first 200 bp from a start codon of the Ryr2 gene; more preferably, the guanine-rich sequence comprises GCAGGGG.

26. A method for regulating binding strength of a T cell to a DC cell, comprising regulating Ryr2 expression.

27. The method according to claim 25, wherein the regulation is a reduction or an improvement; preferably, the method for reducing the binding strength of the T cell to the DC cell comprises improving the Ryr2 expression, or the method for improving the binding strength of the T cell to the DC cell comprises reducing the Ryr2 expression.

28. The method according to claim 26, wherein the reduction of the Ryr2 expression comprises overexpressing FoxP3 in a T cell or adding an Ryr2 inhibitor, the improvement of the Ryr2 expression comprises reducing FoxP3 expression in a T cell or adding an Ryr2 driving agent; wherein the Ryr2 inhibitor is selected from the Ryr2 antagonist according to claim 25, ryanodine, dantrolene or JTV519, the Ryr2 driving agent is selected from nicotinamide adenine dinucleotide phosphate, caffeine, p-chloro-m-cresol, ryanodine, chlorantraniliprole, cyantraniliprole, beta-adrenaline, 4-chloro-3-methylphenol, cyantraniliprole, cyclaniliprole, cyclic adenosine diphosphate ribose, suramin sodium, tetraniliprole or trifluoperazine.

29. The method according to claim 26 or 27, wherein the reduction of the Ryr2 expression comprises knocking down or knocking out the Ryr2 gene in a T cell; preferably, a guanine-rich sequence of the Ryr2 gene in the T cell is knocked down or knocked out; more preferably, the guanine-rich sequence is located within first 200 bp from a start codon of the Ryr2 gene; most preferably, the guanine-rich sequence comprises GCAGGGG.

30. The method according to claim 26 or 27, wherein the reduction of the Ryr2 expression comprises deleting exon 7 of the Ryr2 gene in a T cell.

31. A method for reducing Ryr2 expression in a T cell, reducing basal Ca²⁺ oscillation, reducing m-calpain activity, comprising: overexpressing FoxP3 in the T cell, knocking down or knocking out the Ryr2 gene in the T cell, or adding an Ryr2 inhibitor.

32. A method for improving Ryr2 expression in a T cell, improving basal Ca²⁺ oscillation, improving m-calpain activity, comprising: reducing FoxP3 expression in the T cell, or adding an Ryr2 driving agent.

33. A method for transforming a Tconv cell to having similar function to a Treg cell, comprising overexpressing FoxP3 in the Tconv cell, or knocking down or knocking out Ryr2 gene in the Tconv cell.

34. The method according to claim 32, comprising knocking down or knocking out a guanine-rich sequence of the Ryr2 gene in a Tconv cell; preferably, the guanine-rich sequence is located within first 200 bp from a start codon of the Ryr2 gene; more preferably, the guanine-rich sequence comprises GCAGGGG.

35. The method according to claim 32, comprising knocking out exon 7 of the Ryr2 gene.

36. A method for treating an infectious disease or an inflammation, comprising administering to an individual an effective amount of the T cell according to any one of claims 1 to 5.

37. A method for treating an infectious disease or an inflammation, wherein the method is selected from overexpressing FoxP3 in a T cell of an individual, reducing Ryr2 expression in a T cell of an individual, reducing basal Ca²⁺ oscillation in a T cell of an individual, reducing m-calpain activity in a T cell of an individual, improving binding strength of a T cell of an individual to a DC cell, or adding an Ryr2 inhibitor to an individual.

38. The method according to claim 35 or 36, wherein the infectious disease is selected from a bacterial infection, a viral infection or a fungal infection, preferably selected from a viral infection, pneumonia with septic shock, peritonitis, bacteremia, sepsis or septicemia; the viral infection is selected from an acute viral infection or a chronic viral infection, and the virus is preferably selected from influenza virus, parainfluenza virus, herpes virus, measles virus, vesicular stomatitis virus, hepatitis B virus, hepatitis C virus, human immunodeficiency virus, lymphocytic choriomeningitis virus or human papilloma virus; the inflammation is selected from systemic lupus erythematosus, rheumatoid arthritis, psoriatic arthritis, scleroderma, asthma, atopic dermatitis, organ-specific inflammatory diseases, allergies, folliculitis, tonsillitis, pneumonia, hepatitis, nephritis, acne, autoimmune diseases, chronic prostatitis, glomerulonephritis, hypersensitivity reactions, colitis, inflammatory bowel diseases, pelvic inflammatory disease, reperfusion injury, transplantation rejection, vasculitis or interstitial cystitis.

39. A method for treating a tumor, comprising administering to an individual an effective amount of the T cell according to any one of claims 10 to 11.

40. A method for treating a tumor, wherein the method is selected from reducing FoxP3 expression in a T cell of an individual, improving Ryr2 expression in a T cell of an individual, improving basal Ca²⁺ oscillation in a T cell of an individual, improving m-calpain activity in a T cell of an individual, reducing binding strength of a T cell of an individual to a DC cell, or adding an Ryr2 driving agent to an individual.

41. The method according to claim 38 or 39, wherein the tumor is selected from prostate cancer, breast cancer, liver cancer, glioma, intestinal cancer, cervical cancer, non-small cell lung cancer, lung cancer, pancreatic cancer, gastric cancer, bladder cancer, skin cancer, striated muscle cancer, tongue squamous carcinoma, nasopharyngeal cancer, ovarian cancer, placental choriocarcinoma, lymphoma, leukemia, rectal adenocarcinoma, medulloblastoma, meningioma, neurofibroma, ependymoma, schwannoma, astrocytoma, melanoma, mesothelioma, myeloma, chronic granulocytic leukaemia, acute myelogenous leukemia, myelodysplastic syndrome, chronic lymphocytic leukemia, epidermoid cancer, colon cancer, thymus cancer, blood cancer, head or neck cancer or oropharyngeal cancer.
